(19) Europäisches Patentamt
European Patent Office
Office européen des brevets

(11) EP 1 655 287 A1

(12) EUROPEAN PATENT APPLICATION
published in accordance with Art. 158(3) EPC

(43) Date of publication:
**10.05.2006 Bulletin 2006/19**

(21) Application number: **04771531.3**

(22) Date of filing: **11.08.2004**

(51) Int Cl.:
***C07D 215/12*** *(1985.01)*

(86) International application number:
**PCT/JP2004/011546**

(87) International publication number:
**WO 2005/016888 (24.02.2005 Gazette 2005/08)**

(84) Designated Contracting States:
**AT BE BG CH CY CZ DE DK EE ES FI FR GB GR HU IE IT LI LU MC NL PL PT RO SE SI SK TR**

(30) Priority: **13.08.2003 JP 2003293056**
**28.11.2003 WOPCT/JP03/15229**

(71) Applicant: **BF Research Institute, Inc.**
**Osaka-shi,**
**Osaka 532-8686 (JP)**

(72) Inventors:
- **KUDO, Yukitsuka,**
**Room 300,**
**Kimachi Palu Haitsu**
**Sendai-shi,**
**Miya gi 9800801 (JP)**
- **SUZUKI, Masako**
**5640022 (JP)**
- **SUEMOTO, Takahiro,**
**Room 501,**
**Zefa Koto Maruyamadai**
**Wako-shi Saitama 3510112 (JP)**
- **OKAMURA, Nobuyuki**
**Sendai-shi Miyagi 9800871 (JP)**
- **SHIOMITSU, T.,**
**Rm. 707 Shinosaka Daini Sukaihaitsu**
**Osaka 5320005 (JP)**
- **SHIMAZU, Hiroshi**
**5660024 (JP)**

(74) Representative: **Schnappauf, Georg**
**Dr. Volker Vossius**
**Patent- und Rechtsanwaltskanzlei**
**Geibelstrasse 6**
**81679 München (DE)**

(54) **PROBE FOR DISEASES WITH AMYLOID ACCUMULATION, AMYLOID-STAINING AGENT, REMEDY AND PREVENTIVE FOR DISEASES WITH AMYLOID ACCUMULATION AND DIAGNOSTIC PROBE AND STAINING AGENT FOR NEUROFIBRILLARY CHANGE**

(57) The present invention provides compounds having high affinity for amyloid β-protein which are for the diagnosis of diseases in which amyloid β-protein accumulates, for agents for specifically staining amyloid β-protein, and for the treatment and/or prophylaxis of diseases in which amyloid β-protein accumulates. The present invention also provides probes and staining agents for neurofibrillary tangles.

EP 1 655 287 A1

## Description

### TECHNICAL FIELD

The present invention relates to a probe for the imaging diagnosis of a disease in which amyloid β-protein accumulates, in particular, a probe labeled with a positron-emitting radionuclide, as well as to a composition for imaging diagnosis comprising such a probe. The present invention further relates to detection/staining of, for example, amyloid β-protein in brain samples, for example, senile plaques in the brain of a patient with Alzheimer's disease, and also to a pharmaceutical composition for the prophylaxis and/or treatment of a disease in which β-sheet structure is the cause or possible cause. In addition, the present invention relates to a compound useful for the diagnosis of a disease of which the etiology or an etiology is assigned to neurofibrillary tangles, and to a composition for imaging diagnosis and a composition for staining neurofibrillary tangles, comprising such a compound.

### BACKGROUND ART

Amyloid accumulating diseases include a variety of diseases characterized by the deposition of insoluble fibrillary proteins (amyloids) in various organs and tissues in the body, including Alzheimer's disease, Down's syndrome and others. Among them, Alzheimer's disease (AD) is considered as a disease which is most difficult to treat at present, and there is a need for its accurate and early diagnosis.

Alzheimer's disease (AD) is considered as a disease which is most difficult to treat at present and whose accurate and early diagnosis is required. Alzheimer's disease is a disease whose feature is progressive dementia developing predominantly in a presenile to senile period. Alzheimer's disease is pathologically characterized by overall cerebral atrophy, remarkable degeneration and neuronal loss, and appearing of neurofibrillary tangles and senile plaques. It is known that the highest risk factor of dementia represented by Alzheimer's disease is aging. Thus, an increase in the number of patients as an old population increases is remarkable in especially Japan, America, and European countries, which have reached an aging society, and medical costs for the patients bring the medical system of those countries to a crisis.

In Japan, it is estimated that the number of patients with Alzheimer's disease are about one million, and it is certain that the number of the patients will increase with the aging of the population in future. The costs associated with Alzheimer's disease patients, including the nursing care expense, are supposed to exceed 2.5 million yen per patient for a year, which means that in Japan, socio-economic costs more than 2.5 trillion yen have been already paid for a year. It has now become common in the world that significant effects of medical economy will be brought about by administering treatment before symptoms of dementia in Alzheimer's disease become appeared or as early as possible. At present, however, it is extremely difficult to make an accurate diagnosis of Alzheimer's disease at these stages.

Currently there are various types of methods for diagnosing Alzheimer's disease. Japan commonly employs methods which make a quantitative evaluation of the decrease in cognitive functions of an individual suspected to be affected with Alzheimer's disease, such as Hasegawa's procedure, ADAS, and MMSE, and although not often, imaging diagnosis methods (MRI, CT, and others) are employed supplementarily. However, these methods are insufficient to define the disease, and its definitive diagnosis requires biopsy of the brain before death or histopathological examinations of the brain after death. In spite of these intensive studies on methods for diagnosing Alzheimer's disease, progress has been not made so much. From results of many studies, it has turned out that neural degeneration characteristic of Alzheimer's disease has already took place for a considerable period of time (about 40 years, in the case of a long period) prior to developing its initial clinical symptom. In addition, it is known for Alzheimer's disease that the pathologic feature in the brain has already progressed to an irrecoverable stage when family members and clinicians surrounding an AD patient recognize its initial clinical symptom. Considering together the progression properties of disease conditions and a sharp increase in the number of AD patients as described above, the need for and the value of an accurate, early diagnosis of Alzheimer's disease are of extreme significance.

The histopathological feature of Alzheimer's disease is represented by two major signs: senile plaques and neurofibrillary tangles. The former has, as the main component, amyloid β-protein (Aβ protein) taking β-sheet structures, whereas the latter has, as the main component, hyperphosphorylated amyloid β-protein. The definite diagnosis of Alzheimer's disease relies on the appearance of these pathological characteristics in the brain of a patient.

Amyloid β-protein is characteristic of diseases in which amyloid accumulates, including Alzheimer's disease, and has a close relation with the disease. Thus, detection of amyloid β-protein taking β-sheet structures in the body, especially in the brain, as a marker, will provide for an important method for the diagnosis of a disease in which amyloid accumulates, particularly Alzheimer's disease. In the past, substances which can bind specifically to and stain amyloid β-protein in the body, especially in the brain, have been searched for the purpose of diagnosing diseases in which amyloid accumulates, including Alzheimer's disease. Such substances known include Congo red (non-patent reference 1), thioflavin S (non-patent reference 2), thioflavin T (non-patent reference 3), and chrysamine G and derivatives thereof

(patent references 1, 2), and they have not a few problems in terms of binding specificity to amyloid β-protein, permeability through the blood-brain barrier, solubility, toxicity, and others. The present inventors have found a variety of compounds characterized by having high specificity to amyloid β-protein, high permeability through the blood-brain barrier, high solubilities, reduced toxicities, and others (patent references 3-7).

It is known that intracerebral proteins may take β-sheet structures, thereby resulting in diseases whose etiology can be assigned to such proteins themselves. In the case of Alzheimer's disease, it is supposed that amyloid β-protein and tau protein take β-sheet structures, whereby such proteins themselves are responsible for or contribute to the disease. Yankner et al. have first reported that amyloid β-protein is allowed to take β-sheet structures, thereby displaying neural cytotoxicity (Science, vol. 245, 417-420, 1989). Later, many experiments for corroboration have been performed and ascertained that amyloid β-protein with β-sheet structures possess neural cytotoxicity. Thus, the fact that neural cytotoxicity is observed with amyloid β-protein and tau protein taking β-sheet structures suggests that compounds inhibiting their cytotoxicity could be drugs for treating a disease in which a protein itself takes β-sheet structures, thereby causing or contributing to the disease, for example, Alzheimer's disease.

Until now, studies of Alzheimer's disease or diagnoses employing biopsy or autopsy samples involve preparation of brain sections from a patient with Alzheimer's disease and staining them. Conventional staining agents have mainly utilized Congo red or thioflavin S. These staining agents are characterized by staining both senile plaques and neurofibrillary tangles, which are said to be two major pathological signs of Alzheimer's disease.

However, neither Congo red nor thioflavin S so far has been able to stain diffuse plaques. Also, none of many reports until now describes low molecular-weight organic compounds capable of staining diffuse plaques. It is thought that amyloid β-protein (Aβ protein), which is the major component of senile plaques in Alzheimer's disease, begins its accumulating at a considerably early time (at least 10 years or more) prior to the onset of the disease (developing symptoms of dementia), and that the accumulation feature during this initial period is diffuse plaques. Early detection of diffuse plaques, therefore, will allow an early identification and diagnosis of Alzheimer's disease.

Therefore, there is an increased need for compounds having high specificity to amyloid β-protein which are for the diagnosis of diseases in which amyloid β-protein accumulates, including Alzheimer's disease, for an agent for staining specifically amyloid β-protein, and for the treatment and prophylaxis of diseases in which amyloid β-protein accumulates.

Another histopathological major sign of Alzheimer's disease is neurofibrillary tangles and their main component, hyperphosphorylated tau protein, which are generally supposed to develop later than amyloid β-protein. However, it is likely that neurofibrillary tangles correlate well with the degree of dementia, compared to amyloid β-protein (Braak H and Braak E: Acta Neuropathol., vol. 82, 239-259, 1991; Wischik et al., In "Neurobiology of Alzheimer's Disease," 103-206, Oxford University Press, Oxford, 2001).

Besides Alzheimer's disease, disorders whose major sign is accumulation of tau protein in the brain (tauopathies) include Pick's disease, progressive supranuclear palsy (PSP), and others.

As mentioned above, tau protein is characteristic of diseases having accumulated tau protein, including Alzheimer's disease, and has a close relation with the disease. Thus, detection of tau protein taking β-sheet structures in the body, especially in the brain, as a marker, will provide for an important method for the diagnosis of a disease having accumulated tau, particularly Alzheimer's disease.

Methods have been reported by a few groups for quantifying tau in the body, especially in the cerebrospinal fluid for the purpose of diagnosing diseases having accumulated tau, including Alzheimer's disease (non-patent references 4, 5). However, no probe intended to quantify tau noninvasively in vivo can be found throughout the world.

Therefore, there is an increased need for compounds having high specificity to neurofibrillary tangles which are for the diagnosis and treatment of diseases in which neurofibrillary tangle is the cause or a possible cause, including Alzheimer's disease, or for an agent for staining neurofibrillary tangles.

On the one hand, studies of Alzheimer's disease or diagnoses employing biopsy or autopsy samples until now involve preparation of brain sections from a patient with Alzheimer's disease and staining them. Conventional staining agents have mainly utilized Congo red or thioflavin S. These staining agents are characterized by staining both senile plaques and neurofibrillary tangles, which are said to be two major pathological signs of Alzheimer's disease.

However, none of many reports so far reports low molecular-weight organic compounds capable of staining only neurofibrillary tangles.

Patent reference 1: International Patent Application No. PCT/US96/05918
Patent reference 2: International Patent Application No. PCT/US98/07889
Patent reference 3: Japanese Patent Application No. 2000-080082
Patent reference 4: Japanese Patent Application No. 2000-080083
Patent reference 5: Japanese Patent Application No. 2001-076075
Patent reference 6: International Patent Application No. PCT/JP01/02204
Patent reference 7: International Patent Application No. PCT/JP01/02205
Non-patent reference 1: Puchtler et al., Journal of Histochemistry and Cytochemistry, vol. 10, 35, 1962

Non-patent reference 2: Puchtler et al., Journal of Histochemistry and Cytochemistry, vol. 77, 431, 1983
Non-patent reference 3: LeVine, Protein Science, vol. 2, 404-410, 1993
Non-patent reference 4:Ishiguro et al., Neurosci. Lett., vol. 270, 81-84, 1999
Non-patent reference 5: Itoh et al., Ann. Neurol., vol. 50, 150-156, 2001

DISCLOSURE OF THE INVENTION

PROBLEMS TO BE SOLVED BY THE INVENTION

In view of the above-described circumstances, the present invention provides a substance that has high specificity of binding to amyloid β-protein and an high permeability through the blood-brain barrier and is capable of use as a probe for the imaging diagnosis of a disease in which amyloid β-protein accumulates. In addition, the present invention also provides such a substance which is labeled, for use as a probe for the imaging diagnosis of a disease in which amyloid β-protein accumulates, and a composition and a kit for imaging diagnosis comprising such a probe. The present invention further provides a method for staining amyloid β-protein in brain materials, for example, senile plaques having amyloid β-protein as the main component, and a pharmaceutical composition for the prophylaxis and/or treatment of a disease in which β-sheet structure is the cause or possible cause. In addition, the present invention provides a compound useful for an early diagnosis of Alzheimer's disease or a tauopathy, and a composition for imaging diagnosis and a composition for staining tau, the compositions comprising such a compound.

MEANS TO SOLVE THE PROBLEMS

The present inventors have conducted extensive research, in order to solve the above-described problems. In consequence, the present inventors have found that compounds, or a salt or solvate thereof represented by the formula I have high specificity of binding to amyloid β-protein and furthermore high permeability through the blood-brain barrier, leading to the completion of the present invention. In particular, it can be said that the inventive compounds, which are capable of specifically and clearly staining amyloid β-protein, are compounds allowing an accurate, early diagnosis of, especially, Alzheimer's disease, Down's syndrome, and others. Also, the inventive compounds will allow making a noninvasive diagnosis before death, due to their high permeability through the blood-brain barrier.

Thus, the present invention provides the following:

(1) a compound, or a salt or solvate thereof, which is used as a probe for diagnosing a disease in which amyloid β-protein accumulates, represented by the formula I:

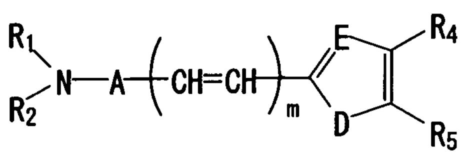

(I)

wherein,
the ring A is a five- or six-membered ring having the following structure:

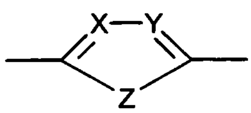

or

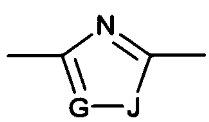

or

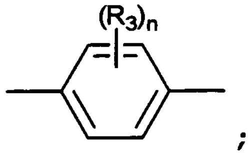

X and Y, independently, are N or CH;
Z is O, S, $CH_2$, or N-$C_pH_{2p+1}$;
G is N or CH;
J is S, O, $CH_2$, or N-$C_qH_{2q+1}$;
p is an integer of 0 to 4;
q is an integer of 0 to 4;
$R_1$ and $R_2$ are independently selected from the group consisting of hydrogen, and alkyl having 1 to 4 carbons (hereinafter, referred to as $C_{1-4}$-alkyl);
$R_3$ is selected from the group consisting of hydrogen, halogen, OH, COOH, $SO_3H$, $NH_2$, $NO_2$, $C_{1-4}$alkyl, and phenyl;
$R_4$ and $R_5$ are independently selected from the group consisting of hydrogen, halogen, OH, COOH, $SO_3H$, $NH_2$, $NO_2$, $C_{1-4}$alkyl, O-$C_{1-4}$alkyl, wherein the $C_{1-4}$alkyl is optionally substituted with halogen(s), and phenyl; or alternatively, $R_4$ and $R_5$, together, form a benzene ring which is optionally substituted with one to four substituents selected form halogen, OH, COOH, $SO_3H$, $NH_2$, $NO_2$, $C_{1-4}$alkyl, O-$C_{1-4}$alkyl, wherein the $C_{1-4}$alkyl is optionally substituted with halogen(s), and phenyl, with the proviso that when m is 0 and when $R_4$ and $R_5$, together, form a benzene ring, the ring A is not a benzene ring;
D is NH, S, O, or CH=CH;
E is N or CH;
m is an integer of 0 to 4, with the proviso that when the ring A is a benzene ring, m is an integer of 2 to 4; and
n is an integer of 0 to 4;

(2) the compound, or a salt or solvate thereof, according to (1), wherein the compound specifically binds to senile plaques and/or diffuse plaques;
(3) the compound, or a salt or solvate thereof, according to (2), wherein the compound is selected from the group consisting of BF-185, BF-187, BF-188, BF-189, BF-196, BF-197, BF-201, BF-214, BF-215, BF-227, and BF-231;
(4) the compound, or a salt or solvate thereof, according to any of (1) to (3), wherein the compound is labeled;
(5) the compound, or a salt or solvate thereof, according to (4), wherein the label is a radionuclide;
(6) the compound, or a salt or solvate thereof, according to (5), wherein the label is a γ-ray emitting nuclide;
(7) the compound, or a salt or solvate thereof, according to (6), wherein the γ-ray emitting nuclide is selected from the group consisting of $^{99m}$TC, $^{111}$In, $^{67}$Ga, $^{201}$Tl, $^{123}$I, and $^{133}$Xe;
(8) the compound, or a salt or solvate thereof, according to (5), wherein the label is a positron emitting nuclide;
(9) The compound, or a salt or solvate thereof, according to (8), wherein the positron emitting nuclide is selected from the group consisting of $^{11}$C, $^{13}$N, $^{15}$O, and $^{18}$F;
(10) a composition for the imaging diagnosis of a disease in which amyloid β-protein accumulates, comprising a compound according to any of (4) to (9), or a pharmaceutically acceptable salt or solvate thereof, and a pharmaceutically acceptable carrier;
(11) a kit for the imaging diagnosis of a disease in which amyloid β-protein accumulates and/or a tauopathy, comprising a compound according to any of (4) to (9), or a pharmaceutically acceptable salt or solvate thereof as the essential ingredient, and a pharmaceutically acceptable carrier;
(12) a composition for staining amyloid β-protein in brain samples, comprising a compound according to any of (1) to (3), or a salt or solvate thereof;
(13) a composition for staining senile plaques and/or diffuse plaques in brain samples, comprising a compound according to (2) or (3), or a salt or solvate thereof;
(14) the composition according to (13), wherein the compound is selected from the group consisting of BF-185 and BF-227;
(15) a pharmaceutical composition for the treatment and/or prophylaxis of a disease in which amyloid β-protein accumulates, comprising a compound according to (1), or a pharmaceutically acceptable salt or solvate thereof, and a pharmaceutically acceptable carrier; and

(16) the pharmaceutical composition according to (15), wherein the disease is Alzheimer's disease.

The present invention further provides the following:

(17) a compound, or a salt or solvate thereof, which is used as a probe for detecting neurofibrillary tangles or as an agent for staining neurofibrillary tangles, represented by the formula II:

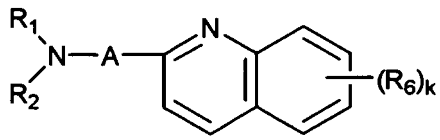

(II)

wherein,
the ring A is represented by

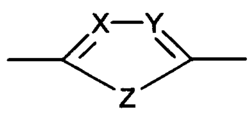

or

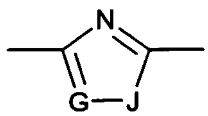

;

$R_1$, $R_2$, X, Y, Z, G, and J are the same as defined above;
$R_6$ is halogen, OH, COOH, $SO_3H$, $NH_2$, $NO_2$, $C_{1-4}$alkyl, O-$C_{1-4}$alkyl, wherein the $C_{1-4}$alkyl is optionally substituted with halogen(s), or phenyl;
k is an integer of 0 to 4; and
(18) the compound according to (17) which is BF-221.

EFFECT OF THE INVENTION

The present invention provides a compound having high specificity to amyloid β-protein, an enhanced property of permeating through the blood-brain barrier, and also extremely high safety. The present invention further provides a compound having high specificity to neurofibrillary tangles (and their main component, tau protein), an enhanced property of permeating through the blood-brain barrier, and also extremely high safety. Thus, the compounds of the present invention would be useful as agents for staining senile plaques and/or diffuse plaques or detecting neurofibrillary tangles in the brain of a patient with Alzheimer's disease. In addition, according to the present invention, there are provided a composition and a kit for the imaging diagnosis of a disease in which amyloid β-protein accumulates or tau protein, wherein the composition and the kit comprise the compound of the present invention. Use of such a compound, composition, or kit will allow making an accurate diagnosis of such a disease at an early stage. Furthermore, according to the present invention, there are also provided a pharmaceutical composition for the prophylaxis and/or treatment of a disease in which accumulation of amyloid β or tau protein is the cause or possible cause, the composition comprising the compound of the present invention, and a method for the prophylaxis and/or treatment of a disease in which accumulation amyloid β or tau protein is the cause or possible cause, the method being characterized by administering the compound of the present invention.

BRIEF DESRIPTION OF THE DRAWINGS

Fig. 1 shows the comparison of staining properties of BSB (upper panel), thioflavin S (middle panel, an adjacent

section of the section in the upper panel), and BF-185 (lower panel, an adjacent section of the middle panel's section) in sections of the brain of a patient with Alzheimer's disease. BF-185 mainly stained senile plaques (wedge-shaped arrowheads), whereas BSB and thioflavin S stained both senile plaques and neurofibrillary tangles (arrowheads).

Fig. 2 shows the comparison of staining properties of BF-185 (left panel) and thioflavin S (right panel, an adjacent section of the left panel's section) in sections of the brain of a patient with Alzheimer's disease. BF-185 mainly stained senile plaques (wedge-shaped arrowheads), whereas thioflavin S stained both senile plaques and neurofibrillary tangles (arrowheads).

Fig. 3 shows the comparison of staining properties of BF-185 (left panel) and immunostaining properties of an anti-Aβ antibody 6F/3D (right panel, an adjacent section of the left panel's section) in sections of the brain of a patient with Alzheimer's disease. BF-185 stained senile plaques (wedge-shaped arrowheads) and diffuse plaques (within dotted-line circles) which were stained with the anti-Aβ antibody 6F/3D.

Fig. 4 shows the comparison of staining properties of BF-187 (left panel) and thioflavin S (right panel, an adjacent section of the left panel's section) in sections of the brain of a patient with Alzheimer's disease. BF-187 stained senile plaques (wedge-shaped arrowheads) which were stained with thioflavin S.

Fig. 5 shows the comparison of staining properties of BF-188 (left panel) and thioflavin S (right panel, an adjacent section of the left panel's section) in sections of the brain of a patient with Alzheimer's disease. BF-188 stained senile plaques (wedge-shaped arrowheads) which were stained with thioflavin S.

Fig. 6 shows the comparison of staining properties of BF-189 (left panel) and thioflavin S (right panel, an adjacent section of the left panel's section) in sections of the brain of a patient with Alzheimer's disease. BF-189 stained senile plaques (wedge-shaped arrowheads) which were stained with thioflavin S.

Fig. 7 shows the comparison of staining properties of BF-196 (left panel) and thioflavin S (right panel, an adjacent section of the left panel's section) in sections of the brain of a patient with Alzheimer's disease. BF-196 mainly stained senile plaques (wedge-shaped arrowheads), whereas thioflavin S stained both senile plaques and neurofibrillary tangles (arrowheads).

Fig. 8 shows the comparison of staining properties of BF-196 (left panel) and immunostaining properties of an anti-Aβ antibody 6F/3D (right panel, an adjacent section of the left panel's section) in sections of the brain of a patient with Alzheimer's disease. BF-196 stained senile plaques (wedge-shaped arrowheads) which were stained with the anti-Aβ antibody 6F/3D.

Fig. 9 shows the comparison of staining properties of BF-197 (left panel) and thioflavin S (right panel, an adjacent section of the left panel's section) in sections of the brain of a patient with Alzheimer's disease. BF-197 mainly stained senile plaques (wedge-shaped arrowheads), whereas thioflavin S stained both senile plaques and neurofibrillary tangles (arrowheads).

Fig. 10 shows the comparison of staining properties of BF-197 (left panel) and immunostaining properties of an anti-Aβ antibody 6F/3D (right panel, an adjacent section of the left panel's section) in sections of the brain of a patient with Alzheimer's disease. BF-197 stained senile plaques (wedge-shaped arrowheads) which were stained with the anti-Aβ antibody 6F/3D.

Fig. 11 shows the comparison of staining properties of BF-201 (left panel) and thioflavin S (right panel, an adjacent section of the left panel's section) in sections of the brain of a patient with Alzheimer's disease. BF-201 stained senile plaques (wedge-shaped arrowheads) which were stained with thioflavin S.

Fig. 12 shows the comparison of staining properties of BF-201 (left panel) and immunostaining properties of an anti-Aβ antibody 6F/3D (right panel, an adjacent section of the left panel's section) in sections of the brain of a patient with Alzheimer's disease. BF-201 stained senile plaques (wedge-shaped arrowheads) which were stained with the anti-Aβ antibody 6F/3D.

Fig. 13 shows the comparison of staining properties of BF-214 (left panel) and thioflavin S (right panel, an adjacent section of the left panel's section) in sections of the brain of a patient with Alzheimer's disease. BF-214 stained senile plaques (wedge-shaped arrowheads) which were stained with thioflavin S.

Fig. 14 shows the comparison of staining properties of BF-215 (left panel) and thioflavin S (right panel, an adjacent section of the left panel's section) in sections of the brain of a patient with Alzheimer's disease. BF-215 stained senile plaques (wedge-shaped arrowheads) which were stained with thioflavin S.

Fig. 15 shows the comparison of staining properties of BF-227 (left panel) and thioflavin S (right panel, an adjacent section of the left panel's section) in sections of the brain of a patient with Alzheimer's disease. BF-227 mainly stained senile plaques (wedge-shaped arrowheads), whereas thioflavin S stained both senile plaques and neurofibrillary tangles (arrowheads).

Fig. 16 shows the comparison of staining properties of BF-227 (left panel) and immunostaining properties of an anti-Aβ antibody 6F/3D (right panel, an adjacent section of the left panel's section) in sections of the brain of a patient with Alzheimer's disease. BF-227 stained senile plaques (wedge-shaped arrowheads) and diffuse plaques (within dotted-line circles) which were stained with the anti-Aβ antibody 6F/3D.

Fig. 17 shows the comparison of staining properties of BF-221 (left panel) and thioflavin S (right panel, an adjacent

section of the left panel's section) in sections of the brain of a patient with Alzheimer's disease. BF-221 stained neurofibrillary tangles (arrowheads) which were stained with thioflavin S.
Fig. 18 shows the comparison of staining properties of BF-221 (left panel) and of an antibody directed to anti-phosphorylated tau (pSer422) (right panel, an adjacent section of the left panel's section) in sections of the brain of a patient with Alzheimer's disease. BF-221 stained neurofibrillary tangles (arrowheads) which were stained with the antibody directed to anti-phosphorylated tau (pSer422).
Fig. 19 shows the comparison of staining properties of BF-231 (left panel) and thioflavin S (right panel, an adjacent section of the left panel's section) in sections of the brain of a patient with Alzheimer's disease. BF-231 stained senile plaques (wedge-shaped arrowheads) which were stained with thioflavin S.

## DETAILED DESCRIPTION OF THE INVENTION

Substances of the present invention which can be used as probes for the imaging diagnosis of diseases in which amyloid β-protein accumulates are compounds, or a salt or solvate thereof, represented by the general formula I as described above. Several examples of the compounds of the formula I are shown in Table 1. Preferable compounds of the present invention are BF-185, BF-187, BF-188, BF-189, BF-196, BF-197, BF-201, BF-214, BF-215, BF-227, BF-231, and others. In particular, BF-185 and BF-227 have high specificity to diffuse plaques and are useful in early identification of Alzheimer's disease. Furthermore, BF-227 is preferable in that it has rapid clearance from the brain.
Among the compounds of the present invention represented by the formula I, compounds represented by the formula II (see, (17) as described above), or a salt or solvate thereof, recognize neurofibrillary tangles well, and are useful as probes recognizing neurofibrillary tangles and as agents for staining neurofibrillary tangles. A typical example of such compounds is BF-221. BF-239, BF-240 and BF-255 also have high selectivity to neurofibrillary tangles.
The compounds of the present invention are of great utility, also from the viewpoint of their extremely low toxicity and high permeability of blood-brain barrier.
In Table 1, thioflavin S, which is known as a compound well recognizing β-structures, is included for reference purpose.

Table 1. Compounds of the present invention specifically recognizing amyloid β-protein

(thioflavin T is included for reference purpose)

| Compound | Degree of β-structure recognition (%) | Structure | |
|---|---|---|---|
| | 61.8 | 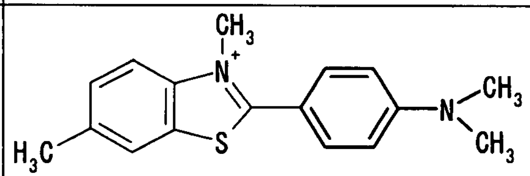 | Thioflavin T |
| BF-185 | 82.7 | 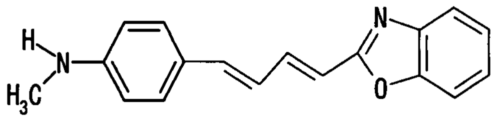 | 2-[[4-(4-methylamino)phenyl]-1,3-butadienyl)benzoxazole |
| BF-187 | 63.3 | 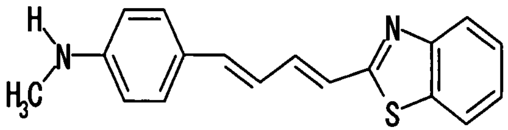 | 2-[[4-(4-methylamino)phenyl]-1,3-butadienyl)benzothiazole |
| BF-188 | 68.9 | 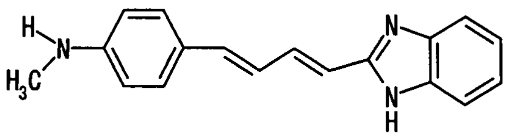 | 2-[[4-(4-methylamino)phenyl]-1,3-butadienyl]benzoimidazole |

| | | | |
|---|---|---|---|
| BF-189 | 79.8 | 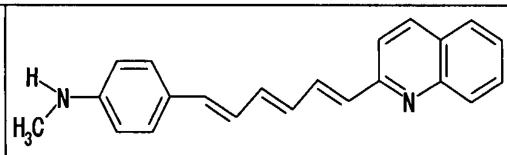 | 1-(4-methylaminophenyl)-6-(2-quinolyl)-1,3,5-hexatrien |
| BF-196 | 53.5 | 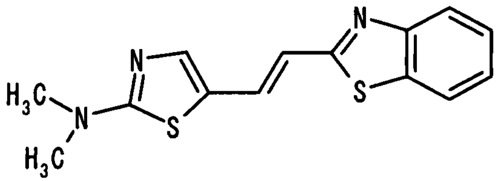 | 2-[2-(2-dimethylaminothiazol-5-yl)ethenyl]benzothiazole |
| BF-197 | 71.8 | 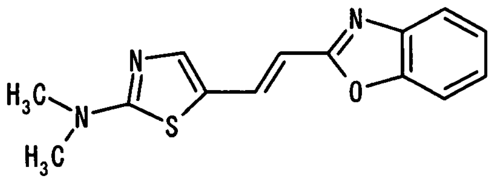 | 2-[2-(2-dimethylaminothiazol-5-yl)ethenyl]benzoxazole |
| BF-201 | 82.0 | 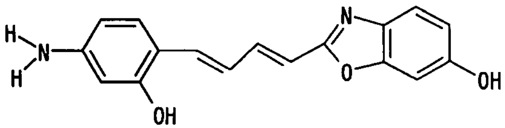 | 2-[4-[(4-amino-2-hydroxy)phenyl]-1,3-butadienyl]-6-hydroxy)benzoxazole |
| BF-214 | 58.9 | 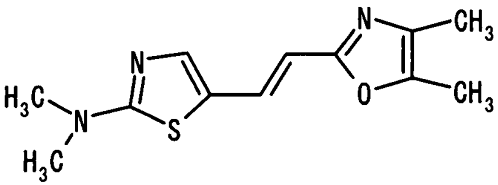 | 2-(dimethylamino)-5-[2-(4,5-dimethyloxazol-2-yl)ethenyl]thiazole |
| BF-215 | 82.6 | 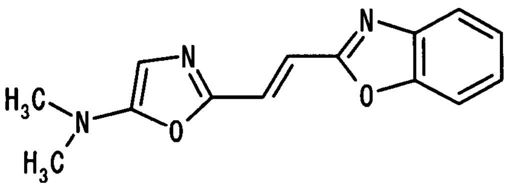 | 2-[2-(5-dimethylaminothiazol-2-yl)ethenyl]benzoxazole |

| | | | |
|---|---|---|---|
| BF-221 | 73.8 | 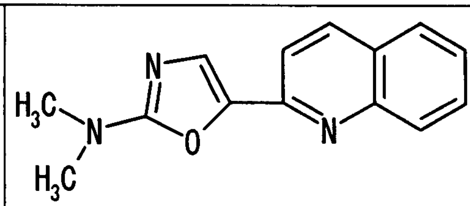 | 2-(2-dimethylaminooxazol-5-yl)quinoline |
| BF-222 | 62.3 | 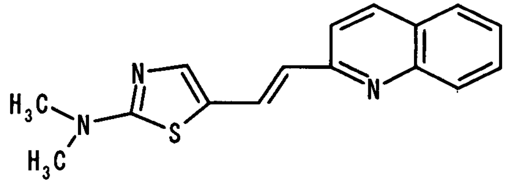 | 2-[2-(2-dimethylaminothiazol-5-yl)ethenyl]quinoline |
| BF-223 | | 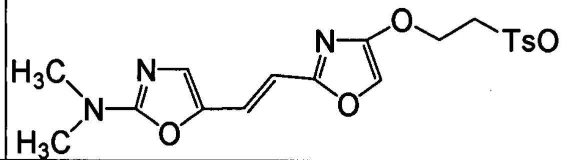 | 2-[2-(2-dimethylaminooxazol-5-yl)ethenyl]-4-[2-(4-tosyloxy)ethoxy]oxazole |
| BF-224 | | 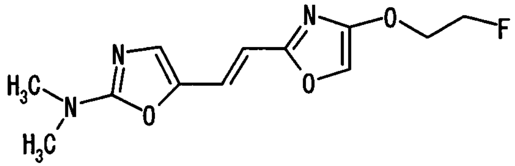 | 2-[2-(2-dimethylaminooxazol-5-yl1)ethenyl]-4-(2-fluoroethyloxy)oxazole |
| BF-225 | 87.5 | 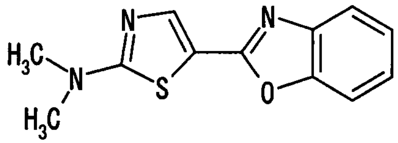 | 2-(2-dimethylaminothiazol-5-yl)benzoxazole |
| BF-226 | 43.3 | 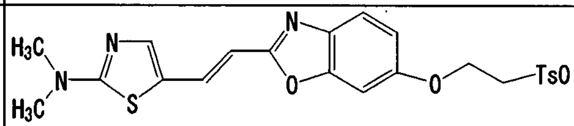 | 2-[2-(2-dimethylaminothiazol-5-yl)ethenyl]-6-[2-(4-tosyloxy)ethoxy]benzoxazole |

| | | | |
|---|---|---|---|
| BF-227 | 70.2 | 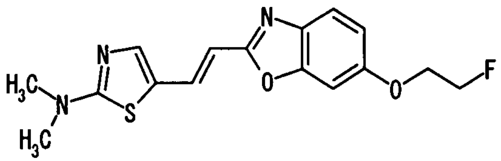 | 2-[2-(2-dimethylaminothiazol-5-yl)ethenyl]-6-(2-fluoroethyloxy)benzoxazole |
| BF-228 | 83.8 | 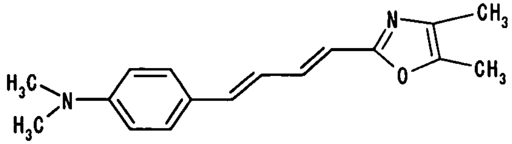 | 2-[4-(4-dimethylaminophenyl)-1,3-butadienyl]-4,5-dimethyloxazole |
| BF-229 | 70.7 | 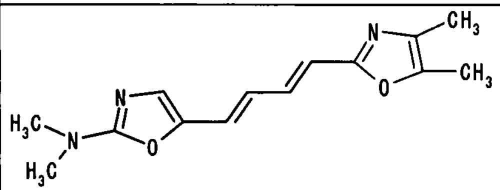 | 2-[4-(2-dimethylaminooxazol-5-yl)-1,3-butadienyl]-4,5-dimethyloxazole |
| BF-230 | 74.0 | 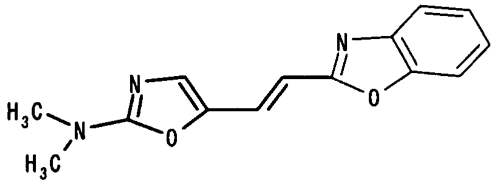 | 2-[2-(2-dimethylaminooxazol-5-yl)ethenyl]benzoxazole |
| BF-231 | 47.2 | 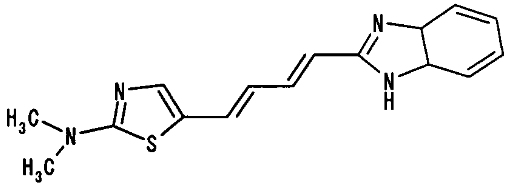 | 2-[4-(2-dimethylaminothiazol-5-yl)-1,3-butadienyl]benzoimidazole |
| BF-232 | | 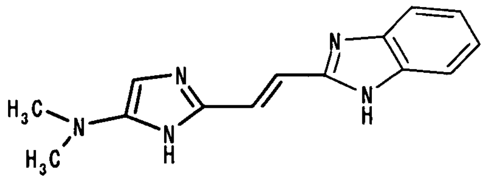 | 2-[2-(4-dimethylaminoimidazol-2-yl)ethenyl]benzoimidazole |

| | | | |
|---|---|---|---|
| BF-233 | | 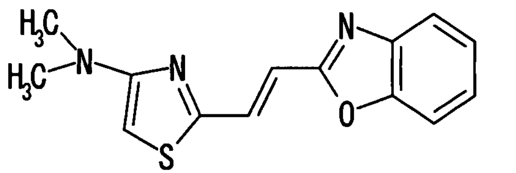 | 2-[2-(4-dimethylaminothiazol-2-yl)ethenyl]benzoxazole |
| BF-235 | 65.5 | 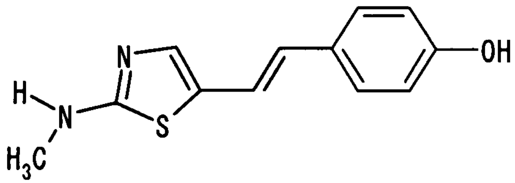 | 5-(4-hydroxystyryl)-2-methylaminothiazole |
| BF-237 | 76.5 | 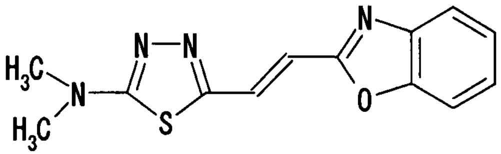 | 2-[2-(2-dimethylamino-1,3,4-thiazol-5-yl)ethenyl]benzoxazole |
| BF-238 | 52.3 | 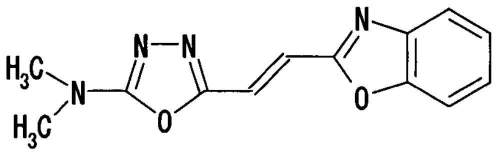 | 2-[2-(2-dimethylamino-1,3,4-oxazol-5-yl)ethenyl]benzoxazole |
| BF-239 | | 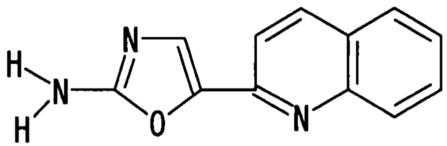 | 2-(2-aminooxazol-5-yl)quinoline |
| BF-240 | 49.9 | 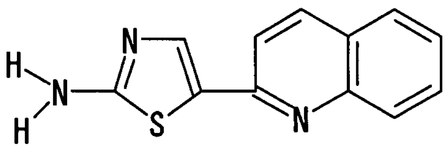 | 2-(2-aminothiazol-5-yl)quinoline |

| | | | |
|---|---|---|---|
| BF-245 | | 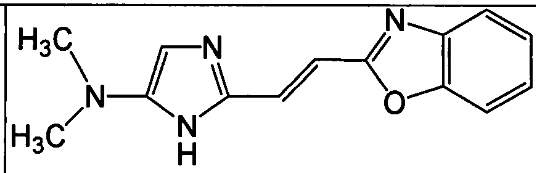 | 2-[2-(5-dimethylaminoimidazol-2-yl)ethenyl]benzoxazole |
| BF-246 | 24.6 | 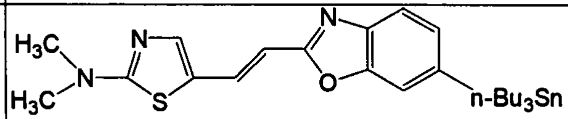 | 6-tri(n-butyl)stannyl-2-[2-(2-dimethylaminothiazol-5-yl)ethenyl]benzoxazole |
| BF-247 | 43.7 | 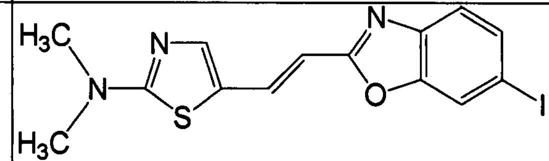 | 6-(iodo)-2-[2-(2-dimethylaminothiazol-5-yl)ethenyl]benzoxazole |
| BF-248 | 40.1 | 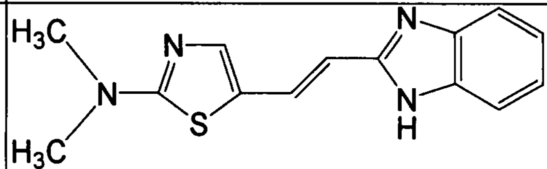 | 2-[2-(2-dimethylaminothiazol-5-yl)ethenyl]benzimidazole |
| BF-250 | 43.1 | 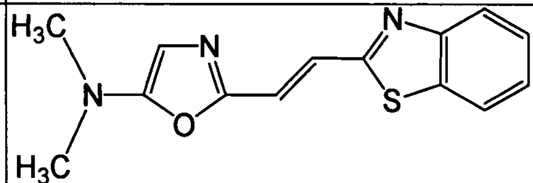 | 2-[2-(5-dimethylaminooxazol-2-yl)ethenyl]benzothiazole |
| BF-251 | 37.5 | 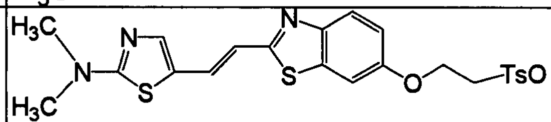 | 2-[2-(2-dimethylaminothiazol-5-yl)ethenyl]-6-[2-(4-tosyloxy)ethoxy]benzothiazole |

| | | | |
|---|---|---|---|
| BF-252 | 47.0 | 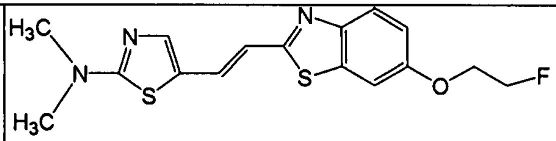 | 2-[2-(2-dimethylaminothiazol-5-yl)ethenyl]-6-[2-(fluoro)ethoxy]benzothiazole |
| BF-253 | | 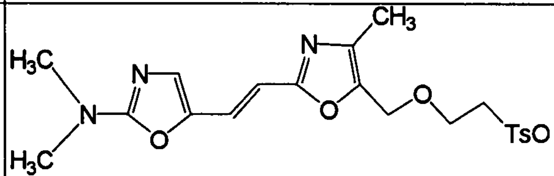 | 2-[2-(2-dimethylaminooxazol-5-yl)ethenyl]-4-methyl-5-[2-(4-tosyloxy)ethoxy]methyloxazole |
| BF-254 | | 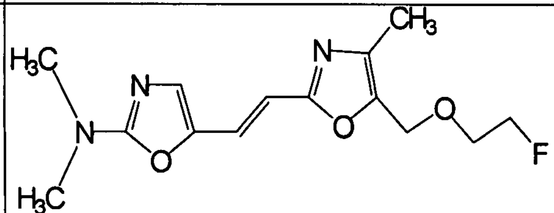 | 2-[2-(2-dimethylaminooxazol-5-yl)ethenyl]-4-methyl-5-(2-fluoroethoxy)methyloxazole |
| BF-255 | 65.8 | 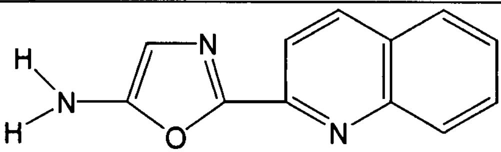 | 2-(5-アミノオキサゾール-2-イル)キノリン |
| BF-256 | | 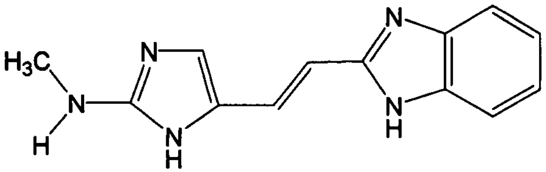 | 2-[2-(2-メチルアミノイミダゾール-5-イル)-エテニル]-ベンズイミダゾール |

| | | | |
|---|---|---|---|
| BF-257 | | 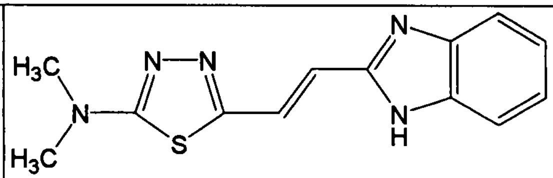 | 2-[2-(2-ジメチルアミノ-1,3,4-チアジアゾール-5-イル)-エテニル]-ベンズイミダゾール |

The following explanation is given of each substituent of the compounds of the formula (I). Further, salts, solvates and derivatives of the compounds of the formula (I), and methods for labeling them are described below. It should be understood that a skilled person in the art can apply these descriptions for the compounds of the formula (I) to the compounds of the formula (II).

As referred to herein, "$C_{1-4}$alkyl" (alkyl having one to four carbons) is intended to include methyl, ethyl, propyl, butyl, and structural isomers thereof. As referred to herein, "halogen" is intended to refer to fluorine, chlorine, bromine, and iodine.

The ring A is a five- or six-membered ring represented by the following structure:

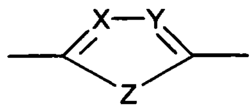

or

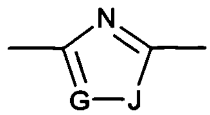

or

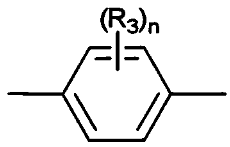

.

The compounds of the present invention in which the ring A is a five-membered ring are preferable in that they have rapid clearance from the brain.

X and Y, independently, are nitrogen (N) or CH.

Z is oxygen (O), sulfur (S), $CH_2$, or N-$C_pH_{2p+1}$. wherein p is an integer of 0 to 4, with the preferable value of p being 1.

G is N or CH.

Jis S, O, $CH_2$, or N-Cp$H_{2q+1}$, wherein q is an integer of 0 to 4, with the preferable value of p being 1.

$R_1$ and $R_2$ are independently selected from the group consisting of hydrogen and $C_{1-4}$alkyl. Preferable examples of $R_1$ and $R_2$ are hydrogen and methyl. $R_1$ and $R_2$ may be the same or different.

$R_3$ is selected from the group consisting of hydrogen, halogen, OH, COOH, $SO_3H$, $NH_2$, $NO_2$, $C_{1-4}$alkyl, and phenyl, with H, OH, and methyl being preferable.

$R_4$ and $R_5$ may be the same or different, and are independently selected from the group consisting of hydrogen, halogen, OH, COOH, $SO_3H$, $NH_2$, $NO_2$, $C_{1-4}$alkyl, phenyl, and O-$C_{1-4}$ alkyl, wherein the $C_{1-4}$alkyl is optionally substituted with halogen(s); or alternatively, $R_4$ and $R_5$, together, form a benzene ring which is optionally substituted with one to four substituents selected form halogen, OH, COOH, $SO_3H$, $NH_2$, $NO_2$, $C_{1-4}$alkyl, and phenyl, with the proviso that when m is 0 and when $R_4$ and $R_5$, together, form a benzene ring, the ring A is not a benzene ring. Preferable $R_4$ and $R_5$ are hydrogen, methyl, and O-methyl or O-ethyl which is optionally substituted with halogen. It is also preferable that $R_4$ and $R_5$, together, form an optionally substituted benzene ring as described above. It is also preferable that such a benzene ring is substituted with OH.

D is NH, S, O, or CH=CH.

E is N or CH.

m is an integer of 0 to 4, with the proviso that when the ring A is a benzene ring, m is an integer of 2 to 4.

n is an integer of 0 to 4.

The preferable value of m is 1 to 3, and the preferable value of n is 0 or 1. In the case of n being other than 0,

$R_3$ substituent(s) may be present at any position of the ring on which the substituent(s) is/are attached. When two or more $R_3$ substituents are present, they may be the same or different.

When a compound has a double bond between the two rings, both cis and trans isomers can exist in such a compound of the formula I.

Also included in the present invention are salts of the compounds of the formula I. Salts may be formed with the nitrogen atom or any functional group within a compound of the formula I. In the case of a compound having a carboxylic or sulfonate group, salts may be formed between the group and metals. Examples of such salts include salts with alkali metals such as lithium, sodium, and potassium, alkaline earth metals such as magnesium, calcium, and barium, and others. In the case of a compound of the formula I having a hydroxyl group, compounds in which the hydrogen atom of a hydroxyl group substituted with metals such as sodium, potassium, or the like are also included in the present invention. In addition, if there are complexes formed between compounds of the formula I and metal salts (for example, complexes with metal salts such as magnesium chloride, iron chloride, and others), these complexes are herein intended to be included in salts of the compounds of the formula I. It is preferable that when a salt of the compound of the formula I is used in the body of a subject, such a salt is a pharmaceutically acceptable salt. Pharmaceutically acceptable salts of the compounds of the formula I include, for example, salts with halide ions such as chlorine, bromine, and iodine, and salts with metals such as sodium, potassium, and calcium. Such salts fall within the present invention. Additionally, solvates of the compounds of the formula I are also included in the present invention. Solvates include hydrates, methanolates, ethanolates, ammoniates, and the like. It is preferable that when a solvate of the compound of the formula I is used in the body of a subject, such a solvate is a pharmaceutically acceptable solvate. Pharmaceutically acceptable solvates include hydrates, ethanolates, and others. In the specification, an "inventive compound" or "compound of the present invention" is intended to include a compound of the formula I (it goes without saying that a compound of the formula II is included), and salts and solvates thereof.

In addition, the present invention also provides a compound which can be used as a precursor for synthesizing the compound of the present invention, that is, the compound represented by the formula I or II. Those skilled in the art are able to design and synthesize such a precursor with ease, based on the structure of the inventive compound of interest. Alternatively, such a precursor may be obtained by modifying a commercially available compound. Precursors of the compounds of the present invention include BF-223 (a precursor of BF-224), BF-226 (a precursor of BF-227), BF-246 (a precursor of BF-247), BF-251 (a precursor of BF-252), BF-253 (a precursor of BF-254), and others.

These precursors are preferably labeled, preferably with radioactive labels. In the synthesis of compounds for PET, they are preferably labeled with $^{18}F$, and in the synthesis of compounds for SPECT, with $^{123}I$. For example, BF-223, BF-226, BF-251, and BF-253 may be labeled with $^{18}F$, whereas BF-246 may be labeled with $^{123}I$.

Accordingly, the present invention provides:

a precursor compound for synthesizing the compound of the formula I or II;

the above-described precursor compound selected from the group consisting of BF-223, BF-226, BF-246, BF-251, and BF-253; and

the above-described precursor compound which is labeled, preferably with $^{18}F$ or $^{123}I$.

For the labeling position and methods, see the explanation regarding the labeling of the compounds of the formula I or II.

The present invention makes use of the compounds of the formula I, or a salt or solvate thereof, which bind specifically to amyloid β-protein (herein also referred to "Aβ protein" or "Aβ") in vivo within the body in a disease in which amyloid β-protein accumulates, as probes for the imaging diagnosis of the disease in which amyloid β-protein accumulates. The inventive compounds allow clear staining of senile plaques. As used herein, a "disease having accumulated Aβ" refers to a disease which has, as the major sign, deposition of Aβ protein in the brain. Diseases whose diagnosis can be made using Aβ protein, i.e., senile plaques, as the marker include Alzheimer's disease, Down's syndrome, and others.

In the diagnosis of a disease in which amyloid β-protein accumulates, compounds of the present invention which have been labeled are generally employed as probes. Labels are fluorescent substances, affinity substances, enzyme substrates, radionuclides, and others. Imaging diagnosis of a disease in which amyloid β-protein accumulates usually uses probes which have been labeled with radionuclides. The inventive compounds can be labeled with a variety of radionuclides by methods well known in the art. For example, $^{3}H$, $^{14}C$, $^{35}S$, $^{131}I$, and others are radionuclides which have been used for a long time, and have many in vitro applications. General requirements for imaging diagnosis probes and means for their detection are to allow making an in vivo diagnosis, to cause less damage to patients (especially, to be non-invasive), to have a high sensitivity of detection, to have an appropriate half-life (to have an appropriate period of time for preparing the labeled probes and for diagnosis), and the like. Accordingly, one have recently tended to employ positron emission tomography (PET) utilizing γ-ray displaying a high sensitivity and permeability of materials or computered tomography (SPECT) with y-ray emitting nuclides. Of them, PET, which detects two γ-rays emitting in opposite directions from a positron emitting nuclide by means of simultaneous counting with a pair of detectors, provides information

which is superior in resolution and quantification and thus is preferable. For SPECT, an inventive compound can be labeled with a γ-ray emitting nuclide, such as $^{99m}$Tc, $^{111}$In, $^{67}$Ga, $^{201}$Tl, $^{123}$I, $^{133}$Xe, and others. $^{99m}$Tc and $^{123}$I are often used for SPECT. For PET, an inventive compound can be labeled with a positron emitting nuclide, such as $^{11}$C, $^{13}$N, $^{15}$O, $^{18}$F, $^{62}$Cu, $^{68}$Ga, $^{76}$Br, and others. Of positron emitting nuclides, $^{11}$C, $^{13}$N, and $^{15}$O are preferable, with $^{18}$F being particularly preferable, from the viewpoint of having an appropriate half-life, the ease of labeling, and the like. The position at which an inventive compound is labeled with a radioactive nuclide, for example, a radiation emitting nuclide such as a positron or γ-ray emitting nuclide, or the like can be any position in the formula I. Alternatively, a hydrogen atom on the benzene ring of an inventive compound may be substituted with a radioactive nuclide such as a positron or γ-ray emitting nuclide. Although the position of labeling the compound of the formula I can be any position as described above, labeling may preferably be carried out at the alkyl group and/or on the phenyl ring of the compound. Such labeled compounds of the formula I are also included in the present invention. For example, when an inventive compound is labeled with $^{18}$F, any position of the side chain may be labeled with $^{18}$F, or a hydrogen on the ring may be substituted with $^{18}$F. Additionally, a hydrogen contained in any of $R_1$ to $R_5$, for example, may be substituted with $^{18}$F or the like.

In general, these nuclides are generated on an instrument termed cyclotron or generator. Those skilled in the art can select methods and instruments for production, depending upon nuclides to be produced. Nuclides thus produced can be used to label the inventive compounds.

Methods for producing labeled compounds, which have been labeled with these radionuclides, are well known in the art. Typical methods include chemical synthesis, isotope exchanging, and biosynthesis processes. Chemical synthesis processes have been traditionally and widely employed, and are essentially the same as usual chemical synthesis processes, except that radioactive starting materials are used. Various nuclides are introduced into compounds by these chemical processes. Isotope exchanging processes are processes by which $^{3}$H, $^{35}$S, $^{125}$I, or the like contained in a compound of a simple structure is transferred into one of a more complex structure, thereby obtaining a compound that has been labeled with the nuclide and possess the more complex structure. Biosynthesis processes are processes by which a compound labeled with $^{14}$C, $^{35}$S, or the like is given to cells such as microorganisms to obtain its metabolites having the nuclide introduced therein.

With respect to the labeling position, similarly to usual synthesis, synthetic schemes can be designed, depending upon the purpose, so that a label can be introduced at a desired position. Such designing is well known to those skilled in the art.

When utilizing positron emitting nuclides, such as $^{11}$C, $^{13}$N, $^{15}$O, and $^{18}$F, which have relatively short half-lives, for example, it is also possible to generate a desired nuclide on a (highly) small-sized cyclotron placed in a facility of hospitals or the like, which in turn is used to label a desired compound at its desired position by any of the above-described methods, followed by carrying out immediately diagnosis, examination, treatment, or the like.

These methods well known to those skilled in art enable one to carry out labeling by introducing a desired nuclide into an inventive compound at its desired position.

The inventive compound which has been labeled may be administered to subjects locally or systemically. Routes for administration include intradermal, intraperitoneal, intravenous, intra-arterial injections or infusions, injections or infusions into the spinal fluid, and others, and can be selected, depending upon factors such as the disease type, nuclide used, compound used, the condition of the subject, the site to be examined, and others. The site to be examined can be investigated with means such as PET, SPECT, or the like by administering an inventive probe, followed by the elapse of a sufficient time to allow its binding to amyloid β-protein and decay. These procedures can be selected as appropriate, depending upon factors such as the disease type, nuclide used, compound used, the condition of the subject, the site to be examined, and others.

The dose of an inventive compound which has been labeled with a radionuclide varies, depending upon the disease type, nuclide used, compound used, the age, physical condition, and gender of the subject, the degree of the disease, the site to be examined, and others. In particular, sufficient care has to be taken about exposure doses to a subject. For example, the amount of radioactivity of an inventive compound labeled with a positron emitting nuclide, such as $^{11}$C, $^{13}$N, $^{15}$O, and $^{18}$F, usually ranges from 3.7 megabecquerels to 3.7 gigabecquerels, and preferably from 18 megabecquerels to 740 megabecquerels.

The present invention also provides a composition for the imaging diagnosis of a disease in which amyloid β-protein accumulates, the composition comprising an inventive compound. The composition of the present invention comprises an inventive compound and a pharmaceutically acceptable carrier. Preferably, the inventive compound in the composition is labeled. Although a variety of labeling methods is possible as described above, labeling with radionuclides (in particular, positron emitting nuclides such as $^{11}$C, $^{13}$N, $^{15}$O, $^{18}$F, and others) is desirable for in vivo image-diagnosis applications. It is preferable from their purposes that the form of the inventive compositions is one allowing injection or infusion. Therefore, pharmaceutically acceptable carriers are preferably liquid and include, but not limiting to, aqueous solvents such as potassium phosphate buffer, saline, Ringer's solution, and distilled water, or non-aqueous solvents such as polyethylene glycols, vegetable oils, ethanol, glycerin, dimethyl sulfoxide, and propylene glycols. The ratio of

formulation of a carrier and an inventive compound can be selected as appropriate, depending upon sites to be applied, means for detection, and the like, and usually ranges from 100,000:1 to 2:1, preferably from 10,000:1 to 10:1. Additionally, the inventive compositions may further contain well-known antimicrobials (for example, antibiotics etc.), local anesthetics (for example, procaine hydrochloride, dibucaine hydrochloride, etc.), buffers (for example, Tris-HCl buffer, HEPES buffer, etc.), osmoregulatory agents (for example, glucose, sorbitol, sodium chloride, etc.), and the like.

Further, the present invention provides a kit for the imaging diagnosis of a disease in which amyloid β-protein accumulates, the kit comprising an inventive compound as the essential ingredient. Usually, the kit is a package in which each of the components such as an inventive compound, solvent for dissolving it, buffer, osmoregulatory agent, antimicrobial, local anesthetic, and the like are packaged separately into respective containers, or some of the components are packaged together into respective containers. The inventive compound may be unlabeled or labeled. When not labeled, the inventive compound can be labeled, prior to use, by usual methods as described above. In addition, the inventive compound may be presented in solid, such as lyophilized powder, or in solutions in appropriate solvents. Solvents may be similar to carriers used in the above-mentioned inventive compositions. Components such as a buffer, an osmoregulatory agent, an antimicrobial, a local anesthetic, and the like, also may be similar to those used in the above-mentioned inventive compositions. While containers can be selected as appropriate, they may be of shapes suitable for carrying out the introduction of a label into an inventive compound, or of light-shielding materials, depending upon the nature of compounds, or take forms such as vials or syringes, so as to be convenient for administration to patients. The kit may also contains, as appropriate, tools necessary for diagnosis, for example, syringes, an infusion set, or apparatus for use in a PET instrument. The kit usually has its instructions attached thereto.

Further, the inventive compounds have properties of binding specifically to amyloid β-protein, and thus can be also used, for example, for staining and quantifying amyloid β-protein in vitro with or without labeling. For example, the inventive compounds can be used for staining amyloid β-protein in microscopic specimens, for colorimetric determination of amyloid β-protein in samples, or for quantifying amyloid β-protein using a scintillation counter.

As mentioned above, Congo red, thioflavin S, and the like stain both amyloid β-protein and neurofibrillary tangles, whereas the inventive compounds have high specificity to amyloid β-protein. Therefore, the inventive compounds are useful, for example, for studies of diseases in which amyloid β-protein accumulates, or in their diagnosis before or after death, and could be useful, for example, as agents for staining senile plaques in the brain of Alzheimer's disease patients. Staining brain sections with the inventive compounds can be carried out in usual methods. In addition, many conventional compounds such as Congo red and thioflavin S cannot stain diffuse plaques. It is believed that the deposition of amyloid β-protein (Aβ protein), which is the main component of senile plaques of Alzheimer's disease, takes place at a much earlier time (at least 10 years earlier) than the disease develops (a dementia symptom becomes appear), and the deposition feature at this early stage would be diffuse plaques. Therefore, early detection of diffuse plaques will allow an early identification/diagnosis of Alzheimer's disease. In that respect, the compounds of the present invention are extremely useful also in an early identification/diagnosis of Alzheimer's disease, because they can provide clear staining of diffuse plaques, as demonstrated in the examples and drawings.

Thus, the present invention is directed to a composition for staining amyloid β-protein in brain samples, the composition comprising an inventive compound, or pharmaceutically acceptable salt or solvate thereof, and to a kit for staining amyloid β-protein in brain samples, the kit comprising an inventive compound, or pharmaceutically acceptable salt or solvate thereof as the essential ingredient. In addition, the present invention is also directed to a method for staining amyloid β-protein in brain samples, the method comprising employing an inventive compound, or pharmaceutically acceptable salt or solvate thereof.

Further, as described above, it has turned out that neural cytotoxicity is observed with amyloid β-protein taking β-sheet structures. Thus, the inventive compounds are likely to become drugs for the treatment of a disease of which the etiology or an etiology is assigned to proteins themselves taking β-sheet structures, such as Alzheimer's disease.

Therefore, the present invention provides:

a method for the treatment and/or prophylaxis of a disease in which amyloid β-protein accumulates, which comprises administering a compound of the formula (I), or a salt or solvate thereof;

a method for the diagnosis of a disease in which amyloid β-protein accumulates, which comprises employing a compound of the formula (I), or a salt or solvate thereof; and

use of a compound of the formula (I), or a salt or solvate thereof, for the production of a composition for the treatment, prophylaxis, or diagnosis of a disease in which amyloid β-protein accumulates.

Diseases in which β-sheet structures is the cause or a possible cause include, for example, Alzheimer's disease, Down's syndrome, and others.

Forms of such pharmaceutical compositions are not limited in particular, but liquid formulations, especially formulations for injection, are preferable. Such formulations for injection can be infused directly into the brain, or alternatively pharmaceutical compositions as described above can be formulated for intravenous injection or drip and administered, since the inventive compounds have high permeability through the blood-brain barrier, as shown in Example 3. Such liquid formulations can be prepared in methods well known in the art. Solutions can be prepared, for example, by dissolving an inventive compound in an appropriate carrier, water for injection, saline, Ringer's solution, or the like, sterilizing the solution through a filter or the like, and filling the sterilized solution into appropriate containers, for example, vials or ampules. Solutions also can be lyophilized and when used, reconstituted with an appropriate carrier. Suspensions can be prepared, for example, by sterilizing an inventive compound, for example, by exposure to ethylene oxide, and then suspending it in a sterilized suspending liquid carrier.

The amount of the inventive compounds to be administered depends on the condition, gender, age, weight of the patient, and the like, and in general ranges from 0.1 mg to 1 g, preferably from 1 mg to 100 mg, more preferably from 5 mg to 50 mg, per day for adult humans weighing 70 kg. It is possible to conduct treatment with such a dosage for a specified period of time, followed by increasing or reducing the dosage according to the outcome.

Further, as described above, among the inventive compounds, compounds represented by the formula II, or a salt or solvate thereof have high specificity to tau protein and can be used as probes recognizing neurofibrillary tangles, or as agents for staining neurofibrillary tangles. Thus, the present invention provides compounds of the formula II, or a salt or solvate thereof, which can be used as probes for imaging diagnosis of neurofibrillary tangles.

Therefore, the present invention provides:

a method for detecting or staining neurofibrillary tangles, which comprises employing a compound of the formula II, or a salt or solvate thereof; and

use of a compound of the formula II, or salt or solvate thereof, for the production of a composition for detecting or staining neurofibrillary tangles.

A preferable compound of the formula II which can be employed for the detection or staining of neurofibrillary tangles as described above is BF-221. Also, BF-239, BF-240 and BF-255 have high selectivity to neurofibrillary tangles.

In addition, the compounds of the present invention, that is, compounds represented by the formula I or II, or a salt or solvate thereof can be used as probes for the diagnosis of a conformational disease, preferably as probes for its imaging diagnosis which are labeled with radiation emitting nuclide. Furthermore, the compounds of the present invention are effective for treatment and/or prophylaxis of a conformational disease. Therefore, the present invention provides:

a compound of the formula I or II, or a salt or solvate thereof, which is used as a probe the diagnosis of a conformational disease;

a composition or kit for the imaging diagnosis of a conformational disease, comprising a compound of the formula I or II, or a salt or solvate thereof;

a pharmaceutical composition for the prophylaxis and/or treatment of a conformational disease, comprising a compound of the formula I or II, or a pharmaceutically acceptable salt or solvate thereof and a pharmaceutically acceptable carrier;

a method for the diagnosis of a conformational disease, which comprises employing a compound of the formula I or II, or a pharmaceutically acceptable salt or solvate thereof;

use of a compound of the formula I or II, or a pharmaceutically acceptable salt or solvate thereof, for the diagnosis of a conformational disease;

a method for the prophylaxis and/or treatment of a conformational disease, which comprises administering to a subject a compound of the formula I or II, or a pharmaceutically acceptable salt or solvate thereof; and

use of a compound of the formula I or II, or a pharmaceutically acceptable salt or solvate thereof, for the prophylaxis and/or treatment of a conformational disease.

Conformational disease include Alzheimer's disease (senile plaques, neurofibrillary tangles), Lewy body disease, Parkinson's disease, Huntington's disease, spinal and bulbar muscular atrophy, dentatorubral-pallidoluysian atrophy, spinocerebellar degeneration, Machado-Joseph disease, amyotrophic lateral sclerosis, Down's syndrome, progressive supranuclear palsy, Pick's disease, FTDP-17 (Frontotemporal Dementia and Parkinsonism Linked to Chromosome 17), LNTD (Limbic Neurofibrillary Tangle Dementia), sudanophilic leukodystrophy, amyloid angiopathy, and others.

The compounds of the present invention can be synthesized from known materials, for example, commercially available materials, by well-known methods. Those skilled in the art are able to select starting materials and synthesis methods, as appropriate, depending on the intended compound of the present invention. The following provides examples of the synthesis of compounds of the present invention, BF-185, BF-196, BF-197, BF-214, BF-225, BF-227, BF-215, and BF-228.

Synthesis of BF-185 (10)

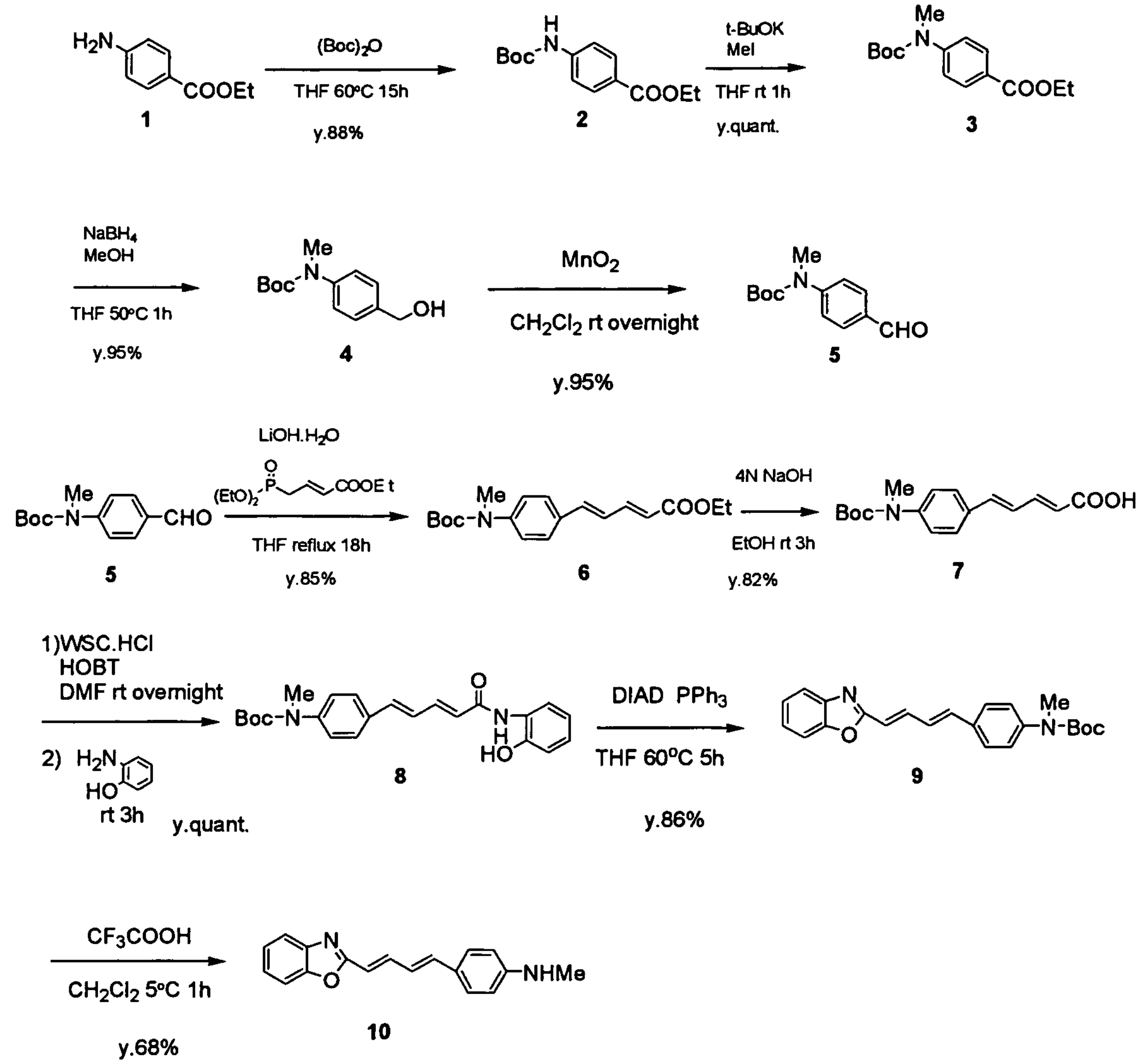


Synthesis of 2:

To tetrahydrofuran (10 ml) were added 1 (5 g, 30 mmol) and $(Boc)_2O$ (9.9 g, 45 mmol), and the mixture was stirred at 60°C for 15 hours. The solvent of the reaction solution was evaporated under reduced pressure. The resulting crystals were recrystallized from ethyl acetate/*n*-hexane to give 2 (7 g, 88%) as colorless crystals.

m.p.: 149-151°C

Synthesis of 3:

Under an argon atmosphere, a solution of 2 (2.9 g, 11 mmol) in tetrahydrofuran (30 ml) was cooled to -60°C, to which potassium t-butoxide (1.5 g, 13 mmol)/tetrahydrofuran (10 ml) was added dropwise over 15 minutes and the mixture was stirred at the same temperature for 1 hour. The reaction mixture was warmed to -20°C, to which a solution of methyl iodide (2.37 g, 17 mmol) in tetrahydrofuran (10 ml) was added dropwise over 5 minutes and the mixture was stirred between - 20°C and room temperature for 1 hour. Ethyl acetate and water were added to the reaction solution and the organic layer was separated. The organic layer was washed with saturated aqueous NaCl solution and dried, and the solvent was evaporated to obtain 3 (3.1 g) as oil, which was used in the next step without further purification.

Synthesis of 4:

To a solution of 3 (3.1 g, 11 mmol) in tetrahydrofuran (10 ml) was added sodium boron hydride (4.2 g, 110 mmol), and the mixture was heated to 50°C, to which methanol (18 ml, 440 mmol) was added dropwise over 1 hour. The reaction solution was cooled on ice, acetone (22 ml) was added to the reaction dropwise over 5 minutes, and the mixture was stirred at the same temperature for 30 minutes. The solvent of the reaction solution was evaporated under reduced pressure. To the residue were added ethyl acetate and water, and the organic layer was separated. The organic layer was washed with water, saturated aqueous NaCl solution and dried. The solvent was evaporated, and the residue was

purified by silica-gel column chromatography (eluting solvent: *n*-hexane:ethyl acetate = 4:1 to 1:1) to give 4 (2.5 g, 95%) as oil.

APCI-MS m/z: 255 $[M+NH_4]^+$

Synthesis of 5:

To a solution 4 (2.1 g, 9 mmol) in methylene chloride (25 ml) was added manganese dioxide (3.9 g, 45 mmol), and the mixture was stirred at room temperature for 18 hours. Insoluble materials were filtered off, and the filtrate was concentrated under reduced pressure. The residue was purified by silica-gel column chromatography (eluting solvent: n-hexane:ethyl acetate = 4:1) to give 5 (5 g, 95%) as oil.

APCI-MS m/z: 253 $[M+NH_4]^+$

Synthesis of 6:

To a solution 5 (1.5 g, 6.4 mmol) in tetrahydrofuran (10 ml) were added triethyl 4-phosphonocrotonate (2.4 g, 9.6 mmol) /tetrahydrofuran (2 ml), synthetic zeolite A-4 beads (8 g), and lithium hydroxide monohydrate (0.4 g, 9.6 mmol), and the mixture was heated under reflux for 18 hours. The reaction solution was cooled on ice and filtered through Celite, and the filtrate was concentrated under reduced pressure. To the residue were added ethyl acetate and water, and the organic layer was separated. The organic layer was washed with water, saturated aqueous NaCl solution, and dried. The solvent was evaporated, and the residue was purified by silica-gel column chromatography (eluting solvent: n-hexane:ethyl acetate = 6:1) to give 6 (1.8 g, 85%) as oil. IR(Neat)2977,2932,1703,1625cm$^{-1}$

APCI-MS m/z: 349 $[M+NH_4]^+$

Synthesis of 7:

To a solution 6 (1.8 g, 5.4 mmol) in ethanol (10 ml) was cooled on ice, to which a 4N solution of sodium hydroxide (2 ml, 8 mmol) was added, and the mixture was stirred at room temperature for 3 hours. The solvent of the reaction solution was evaporated under reduced pressure, and 40 ml of water was added to dissolve the residue, followed by washing with 40 ml of diethyl ether. The aqueous layer was adjusted to a pH of 4 to 5 with 5% citric acid. The resulting solids were collected by filtration, washed with water, and dried under reduced pressure. The solids were recrystallized from ethyl acetate/*n*-hexane to obtain 7 (1.3 g, 82%) as yellow crystals.

m.p.: 172-174°C, IR (Nujol) 2566, 1709, 1679 cm$^{-1}$

APCI-MS m/z: 302 $[M-H]^-$

Synthesis of 8:

To a solution 7 (0.77 g, 2.5 mmol) in dimethyl sulfoxide (15 ml) were added WSC·HCl (0.59 g, 3.0 mmol) and HOBT (0.4 g, 3.0 mmol), and the mixture was stirred at room temperature for 18 hours. A solution of 2-aminophenol (0.83 g, 7.6 mmol) in dimethyl formamide (5 ml) was added to the reaction solution, followed by stirring at room temperature for 3 hours. To the reaction solution were added ethyl acetate and water, and the organic layer was separated. The organic layer was washed with water, saturated aqueous NaCl solution, and dried. The solvent was evaporated, and the residue was purified by silica-gel column chromatography (eluting solvent: *n*-hexane:ethyl acetate = 4:1 to 2:1) to give 8 (1.0 g, 100%) as solids.

APCI-MS m/z: 395 $[M+H]^+$

Synthesis of 9:

To a solution 8 (0.98 g, 2.5 mmol) in tetrahydrofuran (20 ml) were added diisopropyl azodicarboxylate (0.60 g, 3.0 mmol) and triphenylphosphine (0.78 g, 3.0 mmol), and the mixture was stirred at 60°C for 5 hours. The solvent of the reaction solution was evaporated under reduced pressure, and the residue was purified by silica-gel column chromatography (eluting solvent: n-hexane:ethyl acetate = 10:1 to 6:1). The resulting crystals were recrystallized from ethyl acetate/*n*-hexane to give 9 (0.81 g, 86%) as orange crystals.

m.p.: 123-124°C, IR (Nujol) 1694, 1629 cm$^{-1}$

APCI-MS m/z: 377 $[M+H]^+$

Synthesis of 10:

A solution 9 (0.70 g, 1.9 mmol) in methylene chloride (15 ml) was cooled on ice, to which trifluoroacetic acid (2.5 ml) was added, and the mixture was stirred at the same temperature for 1 hour. The reaction solution was poured into a 10% aqueous solution of potassium carbonate (50 ml), and stirred for 5 minutes. After that, 50 ml of ethyl acetate was added, and the organic layer was separated. The organic layer was washed with water, saturated aqueous NaCl solution, and dried. The solvent was evaporated, and the residue was recrystallized in ethyl acetate/*n*-hexane to give 10 (0.35 g, 68%) as red crystals.

m.p.: 143-144°C, IR (Nujol) 3330, 3292, 1583 cm$^{-1}$

APCI-MS m/z: 277 $[M+H]^+$

Synthesis of BF-196 (13)

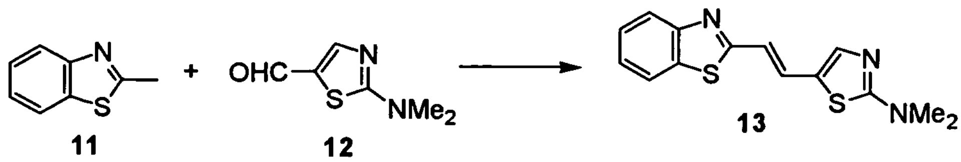

11 12 13

To a solution of 11 (411 mg, 2.75 mmol) and 12 (430 mg, 2.75 mmol) synthesized by the literature method[1)] in dimethyl sulfoxide (3 ml) was added a 50% (w/w) aqueous solution of sodium hydroxide (1.0 ml), with stirring, at room temperature, and the mixture was stirred at room temperature for 1 hour. Water was added to the reaction solution, which was stirred at room temperature for 15 minutes. Precipitates were collected by filtration and washed with water. Crude crystals were recrystallized in ethanol to obtain 13 (554 mg, 70%) as yellow crystals.
m.p.: 158~160°C, IR (Nujol) 1606, 1559 $cm^{-1}$, APCI-MS m/z: 288 $[M+H]^+$

1) Boga, C., Vecchio, E. D., Forlani, L., Todesco, P. E.; J. Organomet. Chem., 2000, 601, 233. and Sawhney, I., Wilson, J. R. H.; J. Chem. Soc. Perkin Trans. 1, 1990, 329.

Synthesis of BF-197 (15)

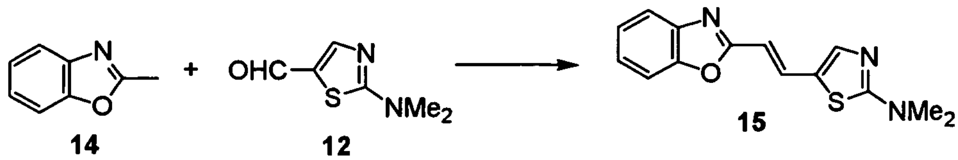

14 12 15

To a solution of 14 (426 mg, 3.20 mmol) and 12 (500 mg, 3.20 mmol) synthesized by the literature method[1)] in dimethyl sulfoxide (3 ml) was added a 50% (w/w) aqueous solution of sodium hydroxide (1.2 ml), with stirring, at room temperature, and the mixture was stirred at room temperature for 1 hour. Water was added to the reaction solution, which was stirred at room temperature for 15 minutes. Precipitates were collected by filtration and washed with water, followed by drying under reduced pressure. Crude crystals were recrystallized in ethanol to obtain 15 (529 mg, 61%) as yellow crystals.
m.p.: 183~185°C, IR (Nujol) 1629, 1581 $cm^{-1}$, APCI-MS m/z: 272 $[M+H]^+$

1) Boga, C., Vecchio, E. D., Forlani, L., Todesco, P. E.; J. Organomet. Chem., 2000, 601, 233. and Sawhney, I., Wilson, J. R. H.; J. Chem. Soc. Perkin Trans. 1, 1990, 329.

Synthesis of BF-214 (18)

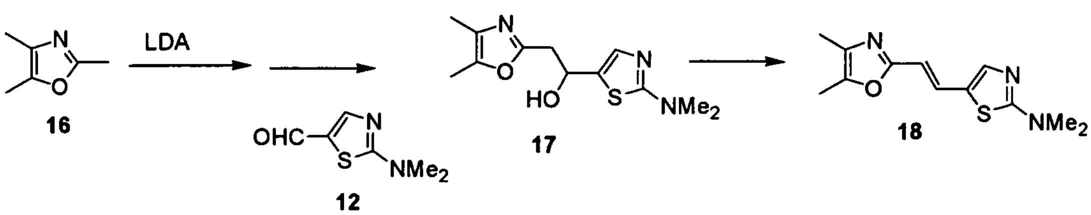


16 12 17 18

Synthesis of 17:
To a solution of diisopropylamine (0.60 ml, 4.23 mmol) in tetrahydrofuran (10 ml) was added dropwise a 1.5M solution of *n*-butyl lithium in n-hexane (2.81 ml, 4.23 mmol) under an argon atmosphere at -60°C or lower, and the temperature was gradually raised to 0°C. Then, a solution of 16 (0.52 ml, 4.23 mmol) in tetrahydrofuran (6 ml) was added dropwise at - 70°C or lower, and the mixture was stirred at -78°C for 1 hour. At the same temperature, a solution of 12 (600 mg, 3.84 mmol) synthesized by the literature method[1)] in tetrahydrofuran (5 ml) was added dropwise, and the mixture was stirred at the same temperature for 30 minutes, at 0°C for 30 minutes, and then at room temperature for 1 hour. Cold water was added to the reaction solution, which in turn was extracted with ethyl acetate. The extract was washed with water and dried, and the solvent was evaporated under reduced pressure. The residue was purified by silica-gel column

chromatography (eluting solvent: *n*-hexane:ethyl acetate = 4/1 to 3/1). To the resulting crystals was added *n*-hexane for trituration, giving 17 (463 mg, 46%) as pale-yellow crystals.
m.p.: 107~112°C, IR (Nujol) 3150, 1649, 1578 $cm^{-1}$, APCI-MS
m/z: 268 $[M+H]^+$

1) Boga, C., Vecchio, E. D., Forlani, L., Todesco, P. E.; J. Organomet. Chem., 2000, 601, 233. and Sawhney, I., Wilson, J. R. H.; J. Chem. Soc. Perkin Trans. 1, 1990, 329.

Synthesis of 18:
To a solution of 17 (463 mg, 1.73 mmol) in dichloromethane (10 ml) was added triethylamine (1.06 ml, 7.62 mmol) and then added methanesulfonyl chloride (0.29 ml, 3.81 mmol) dropwise with cooling on ice while stirring, and the mixture was stirred at the same temperature for 10 minutes, at room temperature for 1 hour. Cold water was added to the reaction solution, which in turn was extracted with ethyl acetate. The extract was washed with water and dried, and the solvent was evaporated under reduced pressure. The residue was purified by silica-gel column chromatography (eluting solvent: n-hexane:ethyl acetate = 5/1 to 2/1), followed by recrystallization in ethyl acetate/*n*-hexane to give 18 (254 mg, 59%) as yellow crystals.
m.p.: 164~166°C, IR (Nujol) 1623, 1573, 1549 $cm^{-1}$, APCI-MS
m/z: 250 $[M+H]^+$

Synthesis of BF-225 (19)

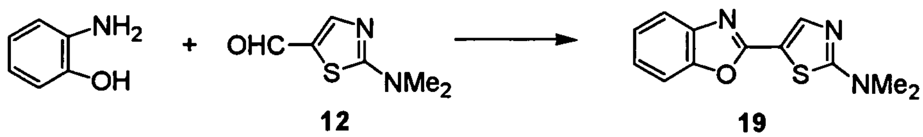

12

19

12 (640 mg, 4.1 mmol) synthesized by the literature method[1)] and 2-aminophenol (469 mg, 4.3 mmol) were stirred without solvent for 2 hour at an external temperature of 150°C. The reaction solution was cooled to room temperature. After that, 40 ml of dioxane and manganese dioxide (3.56 g, 41 mmol) were added, and the mixture was stirred at 100°C for 1 hour. The reaction solution was subjected to hot filtration through Celite, which was washed with warm tetrahydrofuran. The filtrate was concentrated under reduced pressure. The residue was purified by silica-gel column chromatography (eluting solvent: ethyl acetate:*n*-hexane = 4/6), followed by recrystallization in ethyl acetate/*n*-hexane to give 19 (352 mg, 35%) as crystals.
m.p.: 160~161°C, IR (Nujol) 1625, 1565 $cm^{-1}$, APCI-MS m/z: 246 $[M+H]^+$

1) Boga, C., Vecchio, E. D., Forlani, L., Todesco, P. E.; J. Organomet. Chem., 2000, 601, 233. and Sawhney, I., Wilson, J. R. H.; J. Chem. Soc. Perkin Trans. 1, 1990, 329.

Synthesis of BF-227 (24)

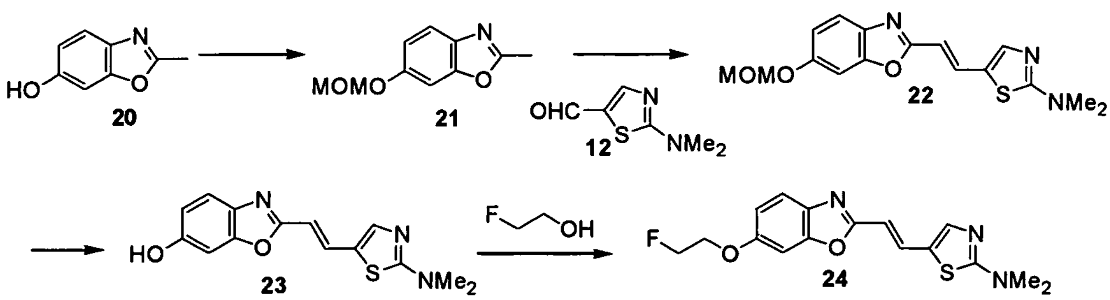

20 21 12 22 23 24

Synthesis of 21:
20 (14.7 g, 98 mmol) synthesized by the literature method[2)] was dissolved in dimethylformamide (80 ml). To this solution, 60% sodium hydride (4.7 g, 118 mmol) was added in portions with cooling on ice while stirring, and then chloromethyl methyl ether (10.0 g, 124 mmol) was added dropwise, and the mixture was stirred at room temperature for 2 hours. Cold water was added to the reaction solution, which was extracted with ethyl acetate. The extract was washed with water and dried, and the solvent was evaporated under reduced pressure. The residue was distilled under reduced pressure

to give 21 (16.7 g, 84%) as colorless oil.
b.p: 133~134°C (7mmHg), IR (Neat) 1619 $cm^{-1}$, APCI-MS m/z: 194 $[M+H]^+$

2) Fujita, S., Koyama, K., Inagaki, Y.; Synthesis, 1982, 68.

Synthesis of 22:
21 (3.18 g, 16.46 mmol) and 12 (2.57 g, 16.46 mmol) synthesized by the literature method[1]) were dissolved in dimethylformamide (18 ml). To this solution, a 50% (w/w) aqueous solution of sodium hydroxide (6.0 ml) was added at room temperature while stirring, and the mixture was stirred at room temperature for 18 hours. Saturated aqueous ammonium chloride solution and water were added to the reaction solution, which was extracted with ethyl acetate. The extract was washed with water and dried, and the solvent was evaporated under reduced pressure. The residue was recrystallized in ethyl acetate/*n*-hexane to give 22 (4.04 g, 74%) as orange crystals.
m.p.: 112~113°C, IR (Nujol) 1625, 1567 $cm^{-1}$, APCI-MS m/z: 332 $[M+H]^+$

1) Boga, C., Vecchio, E. D., Forlani, L., Todesco, P. E.; J. Organomet. Chem., 2000, 601, 233. and Sawhney, I., Wilson, J. R. H.; J. Chem. Soc. Perkin Trans. 1, 1990, 329.

Synthesis of 23:
3) To a solution of 22 (2.53 g, 7.63 mmol) in dichloromethane (25 ml) was added trifluoroacetic acid (17.5 ml) dropwise with cooling on ice while stirring, and the mixture was stirred at room temperature for 40 minutes. The reaction solution was adjusted to a pH of 9 with saturated aqueous sodium hydrogencarbonate solution, followed by extraction with ethyl acetate. The extract was washed with water and dried, and the solvent was evaporated under reduced pressure. The residue was purified by silica-gel column chromatography (eluting solvent: ethyl acetate), followed by recrystallization in ethyl acetate/*n*-hexane to give 23 (1.63 g, 74%) as yellow crystals.
m.p.: 263~264°C, IR (Nujol) 1625, 1575 $cm^{-1}$, APCI-MS m/z: 288 $[M+H]^+$
Synthesis of 24:
4) 23 (800 mg, 2.78 mmol), 2-fluoroethanol (0.359 ml, 6.13 mmol), and triphenylphosphine (1.607 g, 6.13 mmol) were suspended in tetrahydrofuran (20 ml). To the suspension was added diisopropyl azodicarboxylate (1.206 ml, 6.13 mmol) dropwise at room temperature with stirring, and the mixture was stirred at room temperature for 17 hours. The solvent of the reaction solution was evaporated under reduced pressure. The residue was purified by amine-silica-gel column chromatography (Chromatorex® NH; toluene), followed by recrystallization in ethyl acetate/*n*-hexane to give 24 (600 mg, 65%) as yellow crystals.
m.p.: 151~153°C, IR (Nujol) 1631, 1567 $cm^{-1}$, APCI-MS m/z: 334 $[M+H]^+$
Synthesis of BF-215 (31)

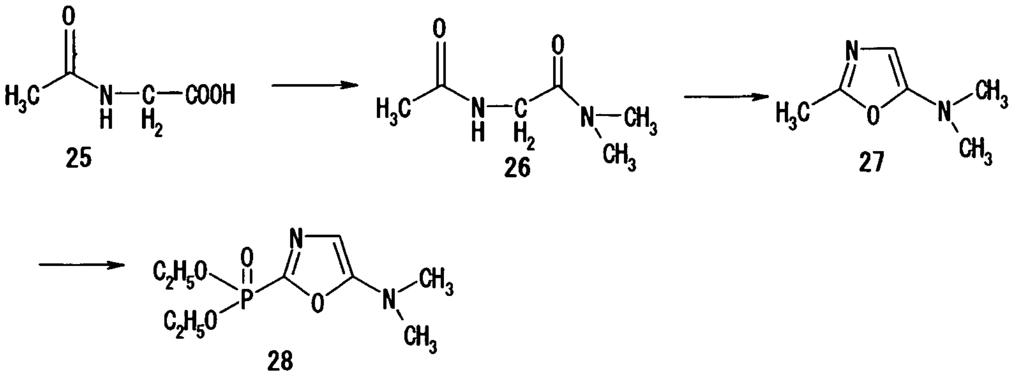

25 26 27 28

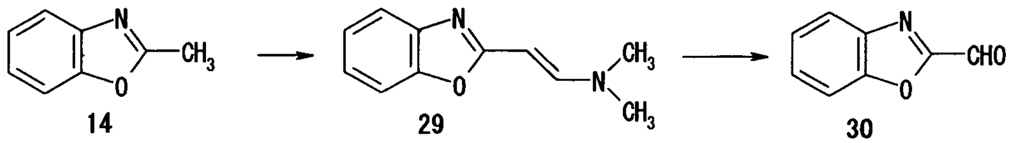

14 29 30

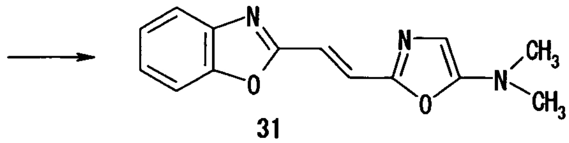

28 + 30 31

Synthesis of 26:

Under an argon stream, DOC (16.5 g, 0.08 mol) and dry methylene chloride (160 ml) were charged into a 300-ml reaction vessel, to which N-acetylglycine (25; 9.4 g, 0.08 mol) was added at room temperature.

The mixture was vigorously stirred at room temperature for 1 hour. Then, dimethylamine (2M in THF; 40 ml, 0.08 mol) was added dropwise over 5 minutes, and the mixture was stirred at room temperature for 21 hours. The reaction mixture was filtered to remove insoluble materials. After the solvent was evaporated, ethyl acetate was added to the residue, and the resulting insoluble material was filtered off. The solvent was evaporated to obtain 8.52 g of 2-acetylamino-N,N-dimethylacetamide (26), which was used in the next step without further purification.

Yield: 73.6%.

Synthesis of 27:

Under an argon stream, triphenylphosphine (10.9 g, 0.042 mol) and dry methylene chloride (87 ml) were charged into a 200-ml reaction vessel, to which a solution of bromine (2.13 ml, 0.042 mol) in dry methylene chloride (18 ml) was added dropwise over 10 minutes at room temperature. After addition, the mixture was stirred at the same temperature for 40 minutes, and then a solution of triethylamine (14.4 ml) and 2-acetylamino-N,N-dimethylacetamide (26; 5.0 g, 0.035 mol) in dry methylene chloride (35 ml) was added at a time. The mixture was then heated under reflux for 1 hour and cooled to room temperature to continue stirring for 3 hours. To the reaction mixture was added *n*-hexane (140 ml), and the resulting crystals was filtered off. The filtrate was concentrated under reduced pressure. The residue was distilled under reduced pre4ssure to give 1.8 g of 5-dimethylamino-2-methyloxazole (27).

Yield: 40.8%.

Synthesis of 28:

Under an argon stream, diisopropylamine (1.6 g, 0.016 mol) and dry THF (20 ml) were charged into a 50-ml reaction vessel and cooled to -60°C. At -50°C or lower, a solution of *n*-butyl lithium in hexane (1.58 mol/L; 10.2 ml) was added dropwise over 10 minutes. After stirring for 15 minutes, a solution of 5-dimethylamino-2-methyloxazole (27; 1.0 g, 0.0079 mol) in dry THF (6 ml) was added dropwise over 45 minutes at -65°C or lower. After addition, stirring was done for 25 minutes, and diethyl chlorophosphate ester (1.4 g, 0.0081 mol) was added dropwise over 25 minutes at -65°C or lower.

The mixture was then stirred at the same temperature for 1 hour. Water (20 ml) was added to the reaction mixture, which was extracted with ethyl acetate, washed with saturated aqueous NaCl solution, dried over anhydrous sodium sulfate. The solvent was evaporated to obtain 1.72 g of 5-dimethylamino-2-[(diethoxyphosphinyl)-methyl]-oxazole (28), which was used in the next step without further purification.
Yield: 83.0%.
Synthesis of 29:
2-Methylbenzoxazole (14; 57.0 g, 0.428 mol), N,N-dimethylformamide dimethylacetal (66.3 g, 0.556 mol), and dry DMF (62.6 g) were charged into a 300-ml autoclave, which was filled with argon gas and heated at 140°C for 72 hours with stirring. The reaction mixture was cooled, and then poured into water/ice (500 ml), which in turn was extracted with ethyl acetate, washed with water, and dried over anhydrous sodium sulfate. The solvent was evaporated, and the resulting residue was washed with *n*-hexane to give 57.7 g of (2-benzoxazol-2-yl-ethenyl)-dimethylamine (29).
Yield: 71.6%.
Synthesis of 30:
(2-Benzoxazol-2-yl-ethenyl)-dimethylamine (29; 6.22 g, 0.03 mol) and THF (80 ml) were charged into a 300-ml reaction vessel, to which water (80 ml) was added. The mixture was cooled to 10°C or lower, and sodium periodate (21.2 g, 0.099 mol) was added. The mixture was stirred at the same temperature for 10 minutes and returned to room temperature to continue stirring for 2 hours. Water (300 ml) was added to the reaction mixture, which was filtered to remove insoluble materials. The organic layer was washed with saturated aqueous sodium hydrogencarbonate solution, saturated aqueous NaCl solution, dried over anhydrous magnesium sulfate. The solvent was evaporated, and the resulting residue was purified on a silica-gel column with *n*-hexane/ethyl acetate = 1/1 to give 0.90 g of benzoxazol-2-carbaldehyde (30).
Yield: 18.5%.
Synthesis of 31:
Under an argon stream, 5-dimethylamino-2-[(diethoxyphosphinyl)-methyl]-oxazole (28; 1.61 g, 0.0061 mol) and dry THF (70 ml) were charged into a 200-ml reaction vessel, to which 60% sodium hydride (in oil; 0.245 g, 0.0061 mol) was added over 10 minutes. After the mixture was stirred for 30 minutes, benzoxazol-2-carbaldehyde (30; 0.9 g, 0.0061 mol) was added over 15 minutes, and then the mixture was stirred for 12 hours. The reaction mixture was poured into water/ice, which in turn was extracted with ethyl acetate, washed with saturated aqueous NaCl solution, dried over anhydrous sodium sulfate. The solvent was evaporated, and the residue was purified on a silica-gel column with *n*-hexane/ethyl acetate = 1/1 to give 620 mg of the first purified product.
Yield: 39.8%.
This product was suspended in and washed with *n*-hexane to give 350 mg of 2-[2-(5-dimethylamino-oxazol-2-yl)ethenyl]-benzoxazole (31).
Yield: 22.5%.
Synthesis of BF-228 (34)

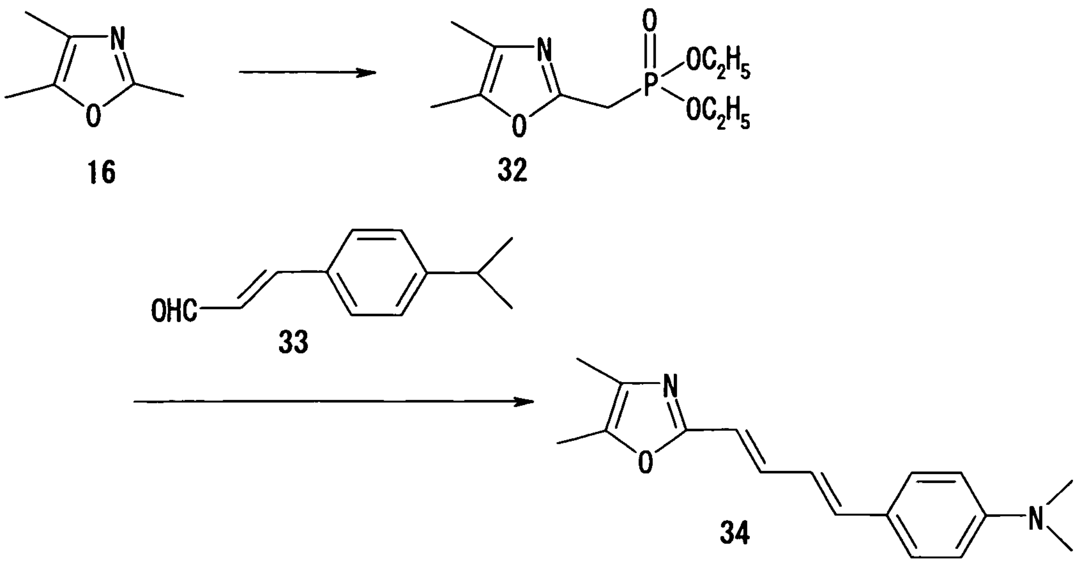

16 32 33 34

Synthesis of 32:

Under an argon stream, diisopropylamine (4.56 g, 0.045 mol) and dry THF (15 ml) were charged into a 100-ml reaction vessel and cooled to -70°C. At -50°C or lower, a solution of *n*-butyl lithium in hexane (1.58 mol/L; 28.5 ml, 0.045 mol) was added dropwise over 15 minutes. After stirring for 80 minutes, a solution of 2,4,5-trimethyloxazole (16; 2.5 g, 0.022 mol) in dry THF (50 ml) was added dropwise over 30 minutes at -65°C or lower. After addition, stirring was done for 30 minutes, and diethyl chlorophosphate ester (3.98 g, 0.023 mol) was added dropwise over 30 minutes at -60°C or lower. The mixture was then stirred at the same temperature for 1 hour. Water (25 ml) was added to the reaction mixture, which was extracted with ethyl acetate, washed with saturated aqueous NaCl solution, dried over anhydrous sodium sulfate. The solvent was evaporated to obtain 2.86 g of 4,5-dimethyl-2-[(diethoxyphosphinyl)-methyl]-oxazole (32).
(Purity: 80%)
This product was used in the next step without further purification.
Yield: 42.1% (based on the purity).
Synthesis of 34:
Under an argon stream, 4,5-dimethyl-2-[(diethoxyphosphinyl)-methyl]-oxazole (2.5 g, 0.0081 mol, based on the purity) and dry THF (70 ml) were charged into a 500-ml reaction vessel, to which 60% sodium hydride (in oil; 0.323 g, 0.0081 mol) was added over 10 minutes. After the mixture was stirred for 10 minutes, 4-dimethylaminocinnamaldehyde (33; 1.42 g, 0.0081 mol) was added over 10 minutes, and then the mixture was stirred for 2 hours. The reaction mixture was poured into water/ice, which in turn was extracted with ethyl acetate, washed with saturated aqueous NaCl solution, dried over anhydrous sodium sulfate. The solvent was evaporated, and the residue was purified on a silica-gel column with *n*-hexane/ethyl acetate = 10/1 to 5/1 to give 940 mg of the first purified product.
Yield: 43.2%.
This product was suspended in and washed with *n*-hexane to give 510 mg of 4,5-dimethyl-2-[4-(4-dimethylaminophenyl)-but-1,3-dienyl]oxazole (34).
Yield: 23.5%.

The Following explanation is made about methods of screening the compounds of the present invention. In the thioflavin T method, some compounds of the present invention overlap with thioflavin T in fluorescence wavelength, making their screening impossible. For these compounds, a new screening protocol as described below was developed.

(1) Amyloid β-protein (purchased from Peptide Institute, Inc.) was dissolved in phosphate buffer (pH 7.4) and allowed to stand at 37°C for 4 days.
(2) 50-μl aliquots of Congo red, dissolved in the same buffer, were placed into wells of a 96-well microplate (at a final concentration of 0.1 μM).
(3) 100 μl of the amyloid β-protein solution was added to each well (at a final concentration of 5 μM) and allowed to stand for 30 minutes.
(4) 100 μl of a test compound, dissolved in the same buffer, was added to wells (at a final concentration of 5 μM) and allowed to stand for 60 minutes.
(5) Measurements were made at the optimal excitation and measurement wavelengths which had been determined, using a fluorescence microplate reader (Molecular Device, Inc., fmax).
(6) The degree of β-structure recognition by the test compound was calculated according to the following formula:

$$\text{the degree of }\beta\text{-structure recognition by a test compound (\%)} = \{(A - B)/(C - D)\} \times 100$$

wherein A denotes a fluorescence in the presence of the test compound, the amyloid β-protein, and Congo red; B denotes a fluorescence in the presence of the test compound and Congo red; C denotes a fluorescence in the presence of the test compound and the amyloid β-protein; and D denotes a fluorescence in the presence of only the test compound.
(7) It can be said that the higher degree of β-structure recognition a test compound has, the higher specificity of binding to amyloid β-protein it has.

The following explanation is made about testing of staining properties of the compounds of the present invention on brain sections of an Alzheimer's disease patient.

(1) Temporal lobe or hippocampus specimens of the brain of a patient who had been definitely diagnosed pathologically as Alzheimer's disease and of a normal aged individual were used. These specimens were provided from Fukushimura Hospital, our joint research facility, with the consent to their use for research purpose received from the bereaved family of the patient (BF Research Laboratories Institutional Ethics Board Permission No. RS-99-02).
(2) The brain tissues embedded in paraffin were sliced 6 or 8 μm thick, extended on slides, and dried. The paraffin-embedded brain sections were deparaffined by washing sequentially with xylene (10 minutes, twice), 100% ethanol (15 minutes, twice), 95% ethanol (15 minutes, twice), and a stream of water (10 minutes).
(3) Pre-treatment for staining with compounds of the present invention involved treating the sections for eliminating self-fluorescence due to lipofuscin. First, the deparaffined sections were immersed into a 10% formalin solution for 60 minutes and washed with PBS for 5 minutes, followed by immersion into a 0.25% solution of $KMnO_4$ for 90 minutes. After washing twice with PBS for 2 minutes each, the sections were immersed into a solution of 0.1% $K_2S_2O_5$/oxalic acid for about 30 seconds each, and then washed three times with PBS for 2 minutes each.
(4) About 150 μl of a 100 μM solution of a compound of the present invention dissolved in 50% ethanol was added dropwise onto the sections and allowed the reaction to be carried out for 10 minutes. The sections were dipped into tap water five times, and then 50% ethanol three to five times, followed by immediate classification. After that, the sections were immersed into PBS for 60 minutes, enclosed with Fluor Save Reagent (Calbiochem), and examined under a fluorescent microscope (Nikon, Eclips E800). Pictures were taken using a digital camera (Plaroid PDMCII).

Immunostaining was carried out as follows.

(a) Procedures for immunostaining amyloid β-protein:

(1) After the sections were deparaffined, the sections were washed twice in distilled water for 2 minutes each, and tissues were marked with an Immunopen. About 150 μl of formic acid was added dropwise and allowed to stand at room temperature for 5 minutes. The sections were washed with tap water for 5 minutes and immersed into cold PBS-Tween 20 for 2 minutes, and then about 150 μl of a 0.05% trypsin solution was added dropwise and allowed the reaction to be carried out at 37°C for 15 minutes.
(2) In an ice bath, the sections were washed twice with cold PBS-Tween 20 for 5 minutes each, and two drops of blocking serum were added and allowed the reaction to be carried out at 37°C for 15 minutes. Excess water was removed, and about 150 μl of 6F/3D, an antibody specific to amyloid β-protein (DAKO; diluted 1:50) was added dropwise and allowed the reaction to be carried out at 37°C for 1 hour.
(3) Further, after washing five times with cold PBS-Tween 20 for 2 minutes each, two drops of a solution of goat antimouse IgG (H+L) conjugated to biotin were added and allowed the reaction to be carried out at 37°C for 1 hour. After that, the sections were washed three times with cold PBS-Tween 20 for 2 minutes each, and two drops of ABS solution (a streptavidin-biotin-peroxidase complex solution) were added and allowed to stand for 30 minutes. The sections were again washed three times with cold PBS-Tween 20 for 2 minutes each, and about 150 μl of DAB solution (prepared by dissolving 10 mg of DAB in 20 ml of 0.05 mol/l Tris-HCl buffer and adding 100 μl of 3% hydrogen peroxide immediately before use) were added and allowed the color to be developed sufficiently. Then, the sections were washed with distilled water for 1 minute to stop the reaction, enclosed, and examined under a microscope.

(b) Procedures for immunostaining neurofibrillary tangles:

(1) Immunostaining of neurofibrillary tangles was carried out as follows. After the sections were deparaffined, the sections were washed twice with distilled water for 2 minutes each, tissues were marked with an Immunopen. About 150 μl of 3% hydrogen peroxide (diluted in methanol) was added dropwise and allowed to stand at room temperature for 10 minutes.
(2) The sections were washed twice with cold PBS-Tween 20 for 5 minutes each, and two drops of blocking serum were added and allowed the reaction to be carried out at 37°C for 30 minutes. Excess water was removed, and two drops of pSer422, an antibody specific to phosphorylated tau (Wako Phosphorylated Tau Immunohistostain Kit 299-57301) and allowed the reaction to be carried out overnight at 4°C.
(3) On the next day, the sections were washed five times with cold PBS-Tween 20 for 2 minutes each, and two drops of a solution of goat anti-rabbit IgG conjugated to biotin were added and allowed the reaction to be carried out at 37°C for 1 hour. After that, the sections were washed three times with cold PBS-Tween 20 for 2 minutes each, and two drops of ABS solution (a streptavidin-biotin-peroxidase complex solution) were added and allowed to stand for 30 minutes.
(4) The sections were again washed three times with cold PBS-Tween 20 for 2 minutes each, and about 150 μl of DAB solution (prepared by dissolving 10 mg of DAB in 20 ml of 0.05 mol/l Tris-HCl buffer and adding 100 μl of 3%

hydrogen peroxide immediately before use) were added and allowed the color to be developed sufficiently. Then, the sections were washed with distilled water for 1 minute to stop the reaction, enclosed, and examined under a microscope. The blocking serum, the solution of goat anti-rabbit IgG conjugated to biotin, and the ABC solution used were those contained in a Phosphorylated Tau Immunohistostain Kit (Wako 299-57301).

The following explanation is made of testing methods for properties of the compounds of the present invention.
(A) Testing of acute toxicity
Acute toxicity of the inventive compounds was determined employing mice by intravenous administration. Male Crj:CD1 mice were used and divided into groups of 4 mice, with an average weight of each group of 31-32 g. Each compound was dissolved in a mixture of 1N HCl, polyethylene glycol 400, and distillated water, or in DMSO, and then diluted with distilled water, and administered via tail vein. Up to 7 days after administration, observations were made.
(B) Testing of blood-brain barrier permeability
Measurements of blood-brain barrier permeability were made according to the following procedures.
Compounds of the present invention were intravenously administered to mice to determine their in vivo blood-brain barrier permeability.

(1) Mice utilized Slc:ICR weighing 30-40 g (7 weeks old, n=3) (Nippon SLC).
(2) A test compound was dissolved in a mixture of 1N HCl, polyethylene glycol 400, and DMSO, or in DMSO or ethyl alcohol, and then diluted with purified water, and injected via tail vein. Two minutes after administration, the mice, under ether anesthesia, were subjected to taking blood from the abdominal aorta with a heparin-treated syringe and removing the brain.
(3) After taking blood, blood samples were centrifuged at 14,000 rpm at 4°C for 10 minutes, and their supernatants were kept at -80°C as plasma sample. The brain (including cerebellum) was kept at -80°C after its removal.
(4) The plasma sample, when used, was thawed, diluted with purified water, and then applied to a conditioned C18 solid-phase extraction cartridge (bond elute C18, 200 mg, Varian), followed by elution with methyl alcohol. Alternatively, after thawing the plasma sample, a diethyl ether/cyclohexane mixture was added, and the mixture was shaken, and then centrifuged to separate an oil layer.
(5) The brain material, when used, was subjected to measuring its wet weight with the brain remaining frozen, and saline was added to the brain for homogenization. The homogenate was centrifuged for 10 minutes, and the supernatant was applied to a conditioned C18 solid-phase extraction cartridge and eluted with methyl alcohol. Alternatively, after measuring the wet weight of the frozen brain, a diethyl ether/cyclohexane mixture was added to the brain, and the mixture was homogenized, shaken, and then centrifuged to separate an oil layer.
(6) The absorbance and fluorescence were detected employing high performance liquid chromatography.
(7) For each of the plasma and brain, the content of the test compound in the plasma or brain (%ID (injected dose) /ml or g) was determined, relative to the administered dose.

## EXAMPLES

Following examples are provided to explain the present invention more specifically, but should not be construed as limiting the present invention thereto in any way.

### Example 1. The inventive compounds are compounds which specifically recognize amyloid β-protein.

According to the above-described method for screening compounds which specifically recognize amyloid β-protein, we have found the compounds listed in Table 1 (in which thioflavin T is included for reference purpose). For the structure of these compounds, see Table 1. The compounds of the present invention tend to display higher degrees of β-structure recognition than thioflavin T (Table 1).
Next, we have examined staining specificity of these compounds in brain sections of a patient with Alzheimer's disease.
The comparison was made of staining properties of BSB ((trans, trans)-1-bromo-2,5-bis-(3-hydroxycarbonyl-4-hydroxy) styrylbenzen) (Fig. 1, upper panel), thioflavin S (Fig. 1, middle panel, an adjacent section of the section in the upper panel), and BF-185 (Fig. 1, lower panel, an adjacent section of the section in the middle panel of Fig. 1) in sections of the brain of a patient with Alzheimer's disease. As shown in Fig. 1, it has turned out that BF-185 mainly stains senile plaques (Fig. 1, wedge-shaped arrowheads), whereas BSB and thioflavin S stains both senile plaques and neurofibrillary tangles (Fig. 1, arrowheads). Staining with BSB was carried out according to the method of D. M. Skovronsky, B. Zhang, M.-P. Kung, H. F. Kung, J. O. Trojanowski, V. M.-Y. Lee, Proc. Natl. Acad. Soc. USA, 97, 7609 (2000).
The comparison was made of staining properties of BF-185 (Fig. 2, left panel) and thioflavin S (Fig. 2, right panel, an adjacent section of the section in the left panel of Fig. 2) in sections of the brain of a patient with Alzheimer's

disease. As shown in Fig. 2, it has turned out that BF-185 mainly stains senile plaques (Fig. 2, wedge-shaped arrowheads), whereas thioflavin S stains both senile plaques and neurofibrillary tangles (Fig. 2, arrowheads).

The comparison was made of staining properties of BF-185 (Fig. 3, left panel) and immunostaining properties of an anti-Aβ antibody 6F/3D (Fig. 3, right panel, an adjacent section of the section in the left panel of Fig. 3,) in sections of the brain of a patient with Alzheimer's disease. As shown in Fig. 3, it has turned out that BF-185 stains senile plaques (Fig. 3, wedge-shaped arrowheads) and diffuse plaques (Fig. 3, within dotted-line circles) which are stained with the anti-Aβ antibody 6F/3D.

The comparison was made of staining properties of BF-187 (Fig. 4, left panel) and thioflavin S (Fig. 4, right panel, an adjacent section of the section in the left panel of Fig. 4) in sections of the brain of a patient with Alzheimer's disease. As shown in Fig. 4, it has turned out that BF-187 stains senile plaques (Fig. 4, wedge-shaped arrowheads) which are stained with thioflavin S.

The comparison was made of staining properties of BF-188 (Fig. 5, left panel) and thioflavin S (Fig. 5, right panel, an adjacent section of the section in the left panel of Fig. 5) in sections of the brain of a patient with Alzheimer's disease. As shown in Fig. 5, it has turned out that BF-188 stains senile plaques (Fig. 5, wedge-shaped arrowheads) which are stained with thioflavin S.

The comparison was made of staining properties of BF-189 (Fig. 6, left panel) and thioflavin S (Fig. 6, right panel, an adjacent section of the section in the left panel of Fig. 6) in sections of the brain of a patient with Alzheimer's disease. As shown in Fig. 6, it has turned out that BF-189 stains senile plaques (Fig. 6, wedge-shaped arrowheads) which are stained with thioflavin S.

The comparison was made of staining properties of BF-196 (Fig. 7, left panel) and thioflavin S (Fig. 7, right panel, an adjacent section of the section in the left panel of Fig. 7) in sections of the brain of a patient with Alzheimer's disease. As shown in Fig. 7, it has turned out that BF-196 mainly stains senile plaques (Fig. 7, wedge-shaped arrowheads), whereas thioflavin S stains both senile plaques and neurofibrillary tangles (Fig. 7, arrowheads).

The comparison was made of staining properties of BF-196 (Fig. 8, left panel) and immunostaining properties of an anti-Aβ antibody 6F/3D (Fig. 8, right panel, an adjacent section of the section in the left panel of Fig. 8) in sections of the brain of a patient with Alzheimer's disease. As shown in Fig. 8, it has turned out that BF-196 stains senile plaques (Fig. 8, wedge-shaped arrowheads) which are stained with the anti-Aβ antibody 6F/3D.

The comparison was made of staining properties of BF-197 (Fig. 9, left panel) and thioflavin S (Fig. 9, right panel, an adjacent section of the section in the left panel of Fig. 9) in sections of the brain of a patient with Alzheimer's disease. As can be seen from Fig. 9, it has turned out that BF-197 mainly stains senile plaques (Fig. 9, wedge-shaped arrowheads), whereas thioflavin S stains both senile plaques and neurofibrillary tangles (Fig. 9, arrowheads).

The comparison was made of staining properties of BF-197 (Fig. 10, left panel) and immunostaining properties of an anti-Aβ antibody 6F/3D (Fig. 10, right panel, an adjacent section of the section in the left panel of Fig. 10) in sections of the brain of a patient with Alzheimer's disease. As shown in Fig. 10, it has turned out that BF-197 stains senile plaques (Fig. 10, wedge-shaped arrowheads) which are stained with the anti-Aβ antibody 6F/3D.

The comparison was made the comparison of staining properties of BF-201 (Fig. 11, left panel) and thioflavin S (Fig. 11, right panel, an adjacent section of the section in the left panel of Fig. 11) in sections of the brain of a patient with Alzheimer's disease. As shown in Fig. 11, it has turned out that BF-201 stains senile plaques (Fig. 11, wedge-shaped arrowheads) which are stained with thioflavin S.

The comparison was made of staining properties of BF-201 (Fig. 12, left panel) and immunostaining properties of an anti-Aβ antibody 6F/3D (Fig. 12, right panel, an adjacent section of the section in the left panel of Fig. 12) in sections of the brain of a patient with Alzheimer's disease. As shown in Fig. 12, it has turned out that BF-201 stains senile plaques (Fig. 12, wedge-shaped arrowheads) which are stained with the anti-Aβ antibody 6F/3D.

The comparison was made of staining properties of BF-214 (Fig. 13, left panel) and thioflavin S (Fig. 13, right panel, an adjacent section of the section in the left panel of Fig. 13) in sections of the brain of a patient with Alzheimer's disease. As can be seen from Fig. 13, it has turned out that BF-214 stains senile plaques (Fig. 13, wedge-shaped arrowheads) which are stained with thioflavin S.

The comparison was made of staining properties of BF-215 (Fig. 14, left panel) and thioflavin S (Fig. 14, right panel, an adjacent section of the section in the left panel of Fig. 14) in sections of the brain of a patient with Alzheimer's disease. As can be seen from Fig. 14, it has turned out that BF-215 stains senile plaques (Fig. 14, wedge-shaped arrowheads) which are stained with thioflavin S.

The comparison was made of staining properties of BF-227 (Fig. 15, left panel) and thioflavin S (Fig. 15, right panel, an adjacent section of the section in the left panel of Fig. 15) in sections of the brain of a patient with Alzheimer's disease. As can be seen from Fig. 15, it has turned out that BF-227 mainly stains senile plaques (Fig. 15, wedge-shaped arrowheads), whereas thioflavin S stains both senile plaques and neurofibrillary tangles (Fig. 15, arrowheads).

The comparison was made of staining properties of BF-227 (Fig. 16, left panel) and immunostaining properties of an anti-Aβ antibody 6F/3D (Fig. 16, right panel, an adjacent section of the section in the left panel of Fig. 16) in sections of the brain of a patient with Alzheimer's disease. It has turned out that BF-227 stains senile plaques (Fig. 16, wedge-shaped arrowheads) and diffuse plaques (Fig. 16, within dotted-line circles) which are stained with the anti-Aβ antibody 6F/3D.

The comparison was made of staining properties of BF-231 (Fig. 19, left panel) and thioflavin S (Fig. 19, right panel, an adjacent section of the section in the left panel of Fig. 19) in sections of the brain of a patient with Alzheimer's disease. As can be seen from Fig. 19, it has turned out that BF-231 stains senile plaques (Fig. 19, wedge-shaped arrowheads) which are stained with thioflavin S.

As conventional agents for staining brain sections of patients with Alzheimer's disease, mainly Congo red or thioflavin S has been used. These staining agents are characterized by staining both senile plaques and neurofibrillary tangles, which are said to be two major pathological signs of Alzheimer's disease.

As shown in Fig. 1, the compounds of the present invention have high specificity to senile plaques and possess specificity different from that of thioflavin S, which stains both senile plaques and phosphorylated amyloid β-protein. From these findings, it is likely that an application of the compounds of the present invention is to use them as staining agents specific to senile plaques in brain sections of patients with Alzheimer's disease.

BF-185 and BF-227, in particular, display affinity also for diffuse plaques and provide their clear staining, and thus are also useful for an early diagnosis of Alzheimer's disease.

Example 2. Acute toxicity testing

Table 2 shows the results of acute toxicity testing on compounds of the present invention performed by the above-described procedures.

Table 2. Results of acute toxicity testing of the inventive compounds

| Compound | Maximum Tolerated Dose (mg/kg, intravenous administration) |
|---|---|
| BF-185 | ≧ 10 |
| BF-187 | ≧ 10 |
| BF-189 | ≧ 10 |
| BF-197 | ≧ 10 |
| BF-201 | ≧ 10 |
| BF-214 | ≧ 10 |
| BF-215 | ≧ 10 |
| BF-222 | ≧ 10 |
| BF-225 | ≧ 10 |
| BF-227 | ≧ 10 |
| BF-228 | ≧ 10 |
| BF-230 | ≧ 10 |
| BF-231 | ≧ 10 |

For PET imaging in humans, in general, total doses of a positron label and an unlabeled compound to be administered utilize single intravenous administrations ranging from $1 \times 10^{-12}$ to $1 \times 10^{-5}$ mg/kg, and often from $1 \times 10^{-10}$ to $1 \times 10^{-7}$ mg/kg. When the comparison is made between the maximum tolerated dose upon intravenous administration of these compounds and the total amount of compounds required for PET imaging, there are at least 100,000 times or more differences between both of these compounds, and therefore the inventive compounds are likely to be compounds which have extremely high levels of safety as probes for PET imaging.

Example 3. Blood-brain barrier permeability

Table 3 shows the permeability of test compounds into the brain in mice two minutes after intravenous administration. The content of the test compounds in the brain two minutes after administration was 3.9 to 19.0%ID/g.
With regard to blood-brain barrier permeability of compounds for PET or SPECT whose target is the central nervous system, it is believed that values of 0.5%ID/g or higher would be sufficient. In that sense, these test compounds are compounds having extremely high levels of blood-brain barrier permeability.

Table 3. Blood-brain barrier permeability of the inventive compounds two minutes after intravenous administration (mice)

| Compound | %ID/g or ml | |
|---|---|---|
| | Brain | Plasma |
| BF-185 | 3.9 | 1.0 |
| BF-187 | 3.6 | 1.3 |
| BF-188 | 4.8 | 1.7 |
| BF-196 | 19.0 | 1.8 |
| BF-197 | 15.0 | 1.9 |
| BF-214 | 8.8 | 2.1 |
| BF-215 | 8.8 | 2.5 |
| BF-222 | 13.0 | 2.0 |
| BF-227 | 7.9 | 2.1 |

Example 4. Neurofibrillary tangles staining by the inventive compounds

As shown in Fig. 17, it has turned out that unlike thioflavin S, BF-221, which is a typical compound of the formula II of the present invention, has high specificity to tau protein (arrowheads). That is, thioflavin S provided enough staining of proteins other than tau protein, and BF-221 did not provide much staining of proteins other than tau protein. Similar results were obtained for BF-240 and BF-255

As shown in Fig. 18, BF-221 stained phosphorylated tau protein which was recognized by an antibody specific to phosphorylated tau protein (pSer422). It has turned out that the compounds represented by the formula II of the present invention could be used as probes or staining agents mainly recognizing tau protein, that is, probes or staining agents recognizing neurofibrillary tangles. Similar results were obtained for BF-239, BF-240 and BF-255.

INDUSTRIAL APPLICABILITY

The compounds of the present invention intended for probes capable of diagnosis of diseases having accumulated Aβ, staining of Aβ using the compounds, pharmaceutical compositions comprising the compounds of the present invention which are used for the treatment and prophylaxis of diseases having accumulated Aβ, and others are extremely important in early identification, medical care, and prophylaxis of serious diseases such as Alzheimer's disease which is one of the most important medical problems and thus have extremely high applicability in the medical field. Also, the present compounds are useful in an early diagnosis of Alzheimer's disease and tauopathies.

## Claims

1. A compound, or a salt or solvate thereof, which is used as a probe for diagnosing a disease in which amyloid β-protein accumulates, represented by the formula I:

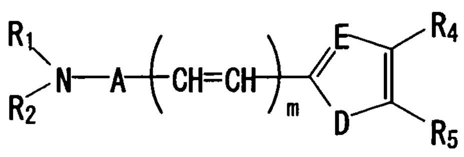

(I)

wherein,
A is a five- or six-membered ring having the following structure:

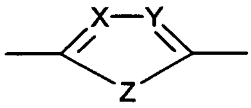

or

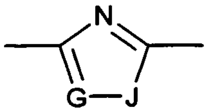

or

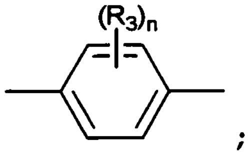

X and Y, independently, are N or CH;
Z is O, S, $CH_2$, or N-$C_pH_{2p+1}$;
G is N or CH;
J is S, O, $CH_2$, or N-$C_qH_{2q+1}$;
p is an integer of 0 to 4;
q is an integer of 0 to 4;
$R_1$ and $R_2$ are independently selected from the group consisting of hydrogen, and alkyl having 1 to 4 carbons (hereinafter, referred to as $C_{1-4}$-alkyl);
$R_3$ is selected from the group consisting of hydrogen, halogen, OH, COOH, $SO_3H$, $NH_2$, $NO_2$, $C_{1-4}$alkyl, and phenyl;
$R_4$ and $R_5$ are independently selected from the group consisting of hydrogen, halogen, OH, COOH, $SO_3H$, $NH_2$, $NO_2$, $C_{1-4}$alkyl, O-$C_{1-4}$alkyl, wherein the $C_{1-4}$alkyl is optionally substituted with halogen(s), and phenyl; or alternatively, $R_4$ and $R_5$, together, form a benzene ring which is optionally substituted with one to four substituents selected form halogen, OH, COOH, $SO_3H$, $NH_2$, $NO_2$, $C_{1-4}$alkyl, O-$C_{1-4}$alkyl, wherein the $C_{1-4}$alkyl is optionally substituted with halogen(s), and phenyl, with the proviso that when m is 0 and when $R_4$ and $R_5$, together, form a benzene ring, the ring A is not a benzene ring;
D is NH, S, O, or CH=CH;
E is N or CH;
m is an integer of 0 to 4, with the proviso that when the ring A is a benzene ring, m is an integer of 2 to 4; and
n is an integer of 0 to 4.

**2.** The compound, or a salt or solvate thereof, according to claim 1, wherein the compound specifically binds to senile plaques and/or diffuse plaques.

**3.** The compound, or a salt or solvate thereof, according to claim 2, wherein the compound is selected from the group consisting of BF-185, BF-187, BF-188, BF-189, BF-196, BF-197, BF-201, BF-214, BF-215, BF-227, and BF-231.

**4.** The compound, or a salt or solvate thereof, according to any one of claims 1 to 3, wherein the compound is labeled.

**5.** The compound, or a salt or solvate thereof, according to claim 4, wherein the label is a radionuclide.

**6.** The compound, or a salt or solvate thereof, according to claim 5, wherein the label is a $\gamma$-ray emitting nuclide.

**7.** The compound, or a salt or solvate thereof, according to claim 6, wherein the $\gamma$-ray emitting nuclide is selected from

the group consisting of $^{99m}Tc$, $^{111}In$, $^{67}Ga$, $^{201}Tl$, $^{123}I$, and $^{133}Xe$.

**8.** The compound, or a salt or solvate thereof, according to claim 5, wherein the label is a positron emitting nuclide.

**9.** The compound, or a salt or solvate thereof, according to claim 8, wherein the positron emitting nuclide is selected from the group consisting of $^{11}C$, $^{13}N$, $^{15}O$, and $^{18}F$.

**10.** A composition for the imaging diagnosis of a disease in which amyloid β-protein accumulates, comprising a compound according to any one of claims 4 to 9, or a pharmaceutically acceptable salt or solvate thereof, and a pharmaceutically acceptable carrier.

**11.** A kit for the imaging diagnosis of a disease in which amyloid β-protein accumulates, comprising a compound according to any one of claims 4 to 9, or a pharmaceutically acceptable salt or solvate thereof as the essential ingredient, and a pharmaceutically acceptable carrier.

**12.** A composition for staining amyloid β-protein in brain samples, comprising a compound according to any one of claims 1 to 3, or a salt or solvate thereof.

**13.** A composition for staining senile plaques and/or diffuse plaques in brain samples, comprising a compound according to claim 2 or 3, or a salt or solvate thereof.

**14.** The composition according to claim 13, wherein the compound is selected from the group consisting of BF-185 and BF-227.

**15.** A pharmaceutical composition for the treatment and/or prophylaxis of a disease in which amyloid β-protein accumulates, comprising a compound according to claim 1, or a pharmaceutically acceptable salt or solvate thereof, and a pharmaceutically acceptable carrier.

**16.** The pharmaceutical composition according to claim 15, wherein the disease is Alzheimer's disease.

**17.** A compound, or a salt or solvate thereof, which is used as a probe for detecting neurofibrillary tangles or as an agent for staining neurofibrillary tangles, represented by the formula II:

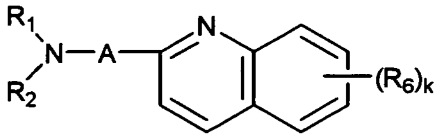

(II)

wherein,
the ring A is represented by

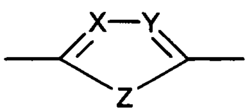

or

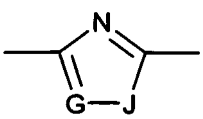

;

$R_1$, $R_2$, X, Y, Z, G, and J are the same as defined above;
$R_6$ is halogen, OH, COOH, $SO_3H$, $NH_2$, $NO_2$, $C_{1-4}$alkyl, O-$C_{1-4}$alkyl,
wherein the $C_{1-4}$alkyl is optionally substituted with halogen(s), or phenyl; and
k is an integer of 0 to 4.

**18.** The compound according to claim 17, which is selected from the group consisting of BF-221, BF-239, BF-240 and BF-255.

**19.** A method for the treatment and/or prophylaxis of a disease in which amyloid β-protein accumulates, which comprises administering a compound according to claim 1, or salt or solvate thereof.

**20.** A method for the diagnosis of a disease in which amyloid β-protein accumulates, which comprises employing a compound according to claim 1, or a salt or solvate thereof.

**21.** Use of a compound according to claim 1, or a salt or solvate thereof for producing a composition for the treatment, prophylaxis, or diagnosis of a disease in which amyloid β-protein accumulates.

**22.** A method for detecting or staining neurofibrillary tangles, which comprises employing a compound according to claim 17, or a salt or solvate thereof.

**23.** Use of a compound according to claim 17, or salt or solvate thereof for producing a composition for detecting or staining neurofibrillary tangles.

**24.** The method according to claim 22 or the use according to claim 23, wherein the compound is selected from the group consisting of BF-221, BF-239, BF-240 and BF-255.

**25.** The compound, or a salt or solvate thereof, according to any one of claims 1 to 9, 17, and 18, which is used as a probe for the diagnosis of a conformational disease.

**26.** A composition or kit for the imaging diagnosis of a conformational disease, comprising a compound, or a salt or solvate thereof, according to any one of claims 1 to 9, 17, and 18.

**27.** A pharmaceutical composition for the prophylaxis and/or treatment of a conformational disease, comprising a compound according to any one of claims 1 to 9, 17, and 18, or a pharmaceutically acceptable salt or solvate thereof and a pharmaceutically acceptable carrier.

**28.** A method for the diagnosis of a conformational disease, which comprises employing a compound according to any one of claims 1 to 9, 17, and 18, or a pharmaceutically acceptable salt or solvate thereof.

**29.** Use of a compound according to any one of claims 1 to 9, 17, and 18, or a pharmaceutically acceptable salt or solvate thereof, for the diagnosis of a conformational disease.

**30.** A method for the prophylaxis and/or treatment of a conformational disease, which comprises administering to a subject a compound according to any one of claims 1 to 9, 17, and 18, or a pharmaceutically acceptable salt or solvate thereof.

**31.** Use of a compound according to any one of claims 1 to 9, 17, and 18, or a pharmaceutically acceptable salt or solvate thereof, for the prophylaxis and/or treatment of a conformational disease.

**32.** A precursor compound for synthesizing a compound according to any one of claims 1 to 9, 17, and 18.

**33.** The precursor compound according to claim 32, which is selected from the group consisting of BF-223, BF-226, BF-246, BF-251, and BF-253.

**34.** The precursor compound according to claim 32 or 33, which is labeled.

**35.** The precursor compound according to any one of claims 32 to 34, which is labeled with $^{18}F$ or $^{123}I$.

[FIG. 1]

BSB STAINING

THIOFLAVIN S STAINING

BF-185 STAINING

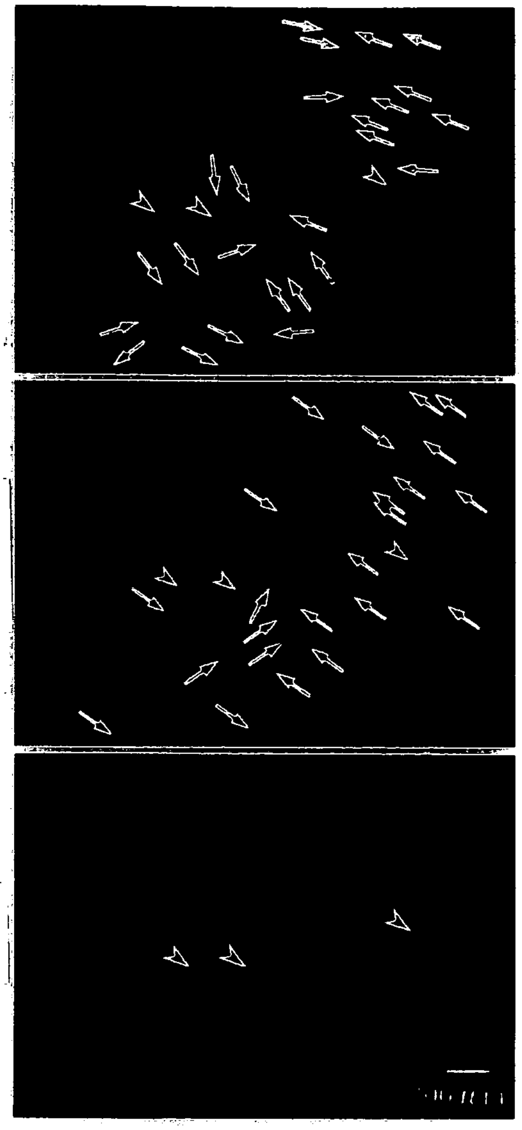

[FIG. 2]

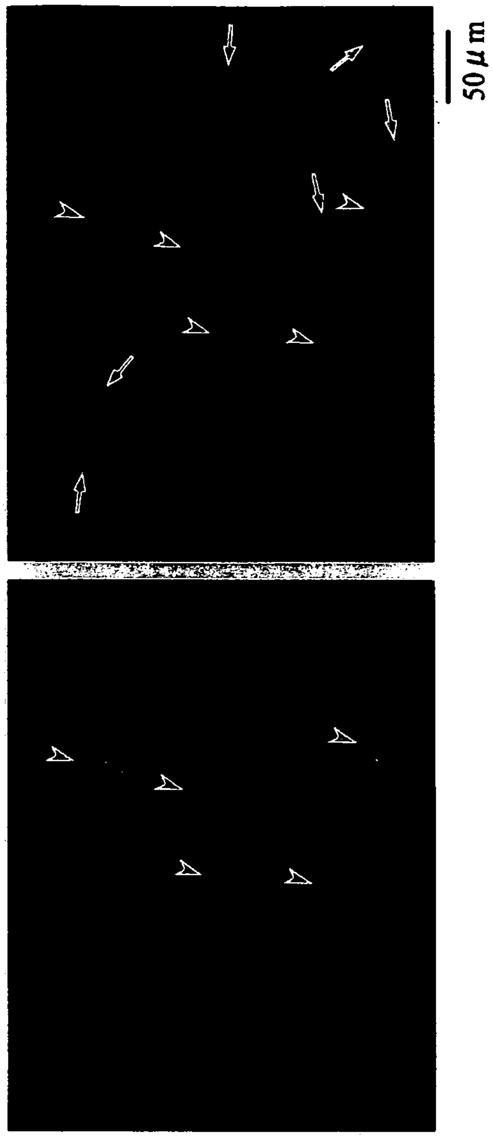


THIOFLAVIN S STAINING

BF-185 STAINING

[FIG. 3]

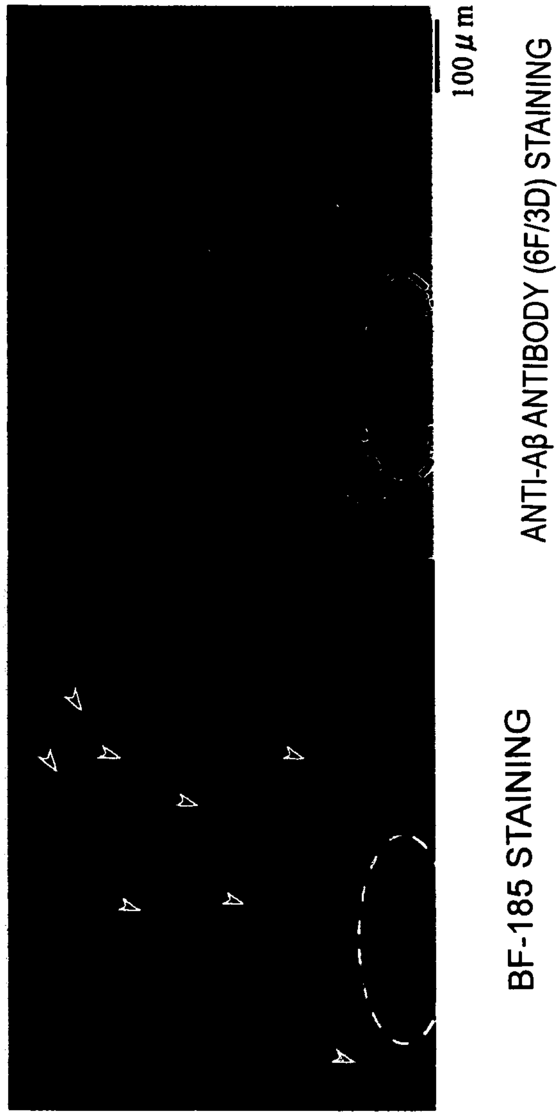

[FIG. 4]

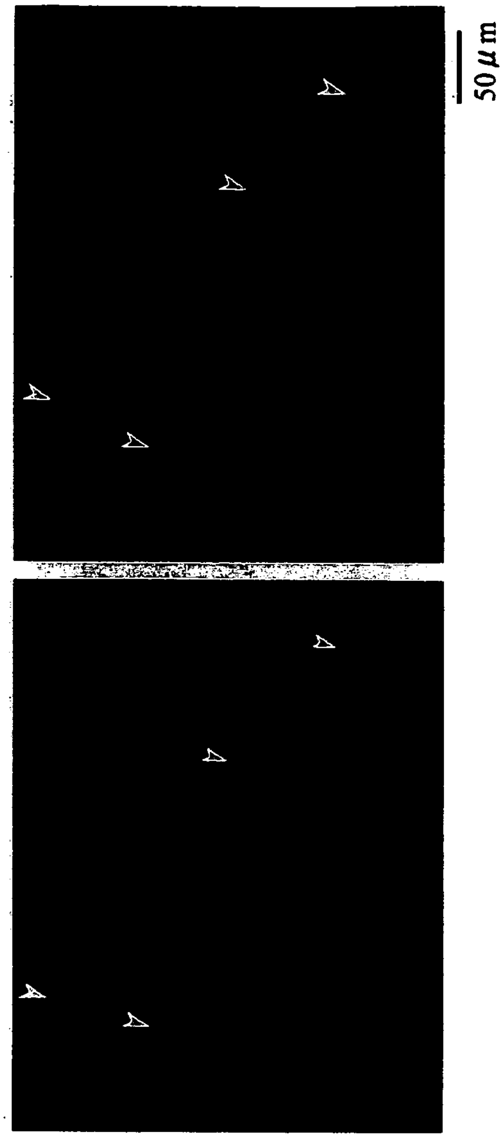


THIOFLAVIN S STAINING

BF-187 STAINING

[FIG. 5]

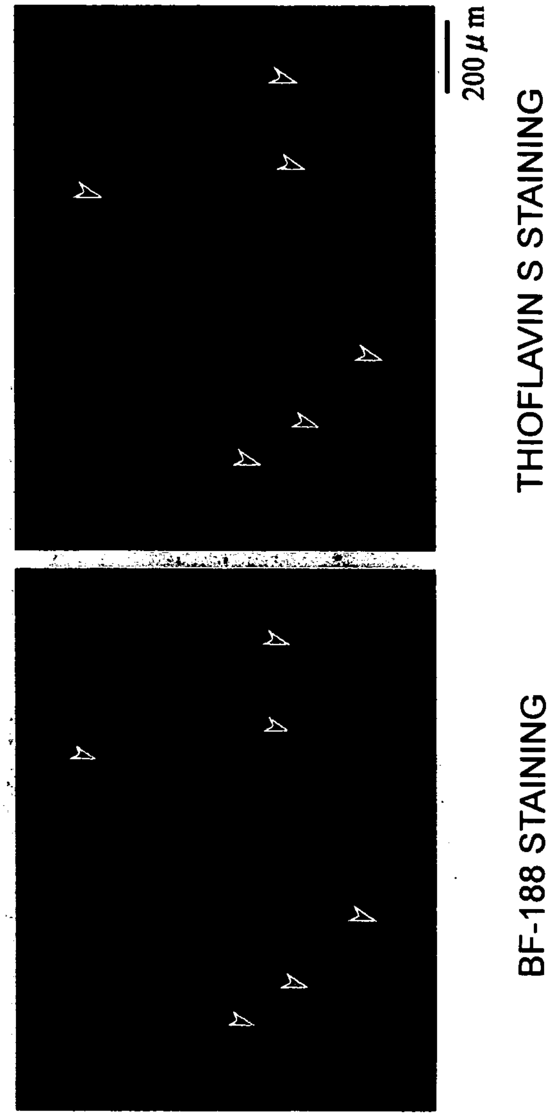

[FIG. 6]

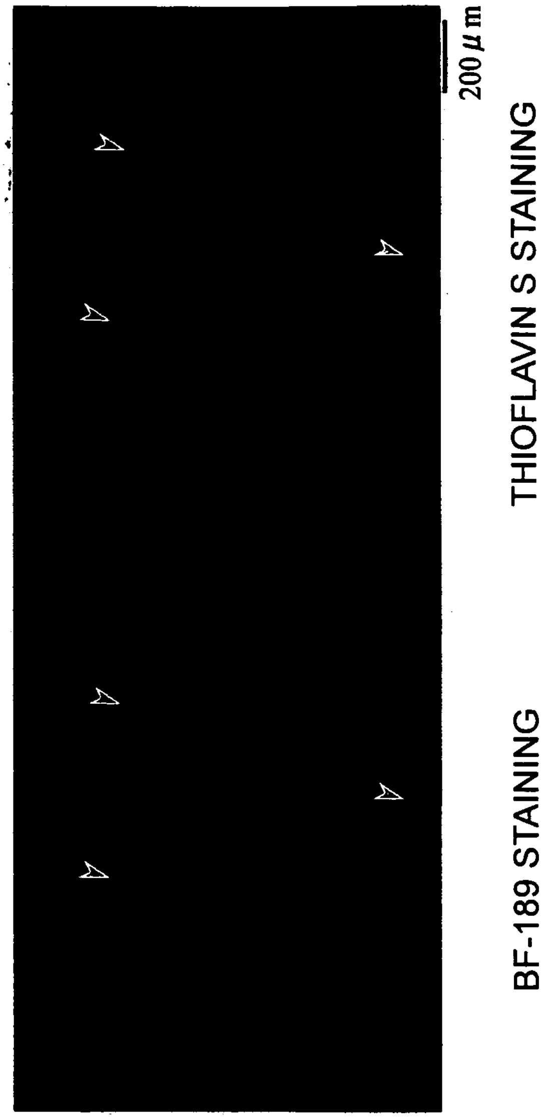

[FIG. 7]

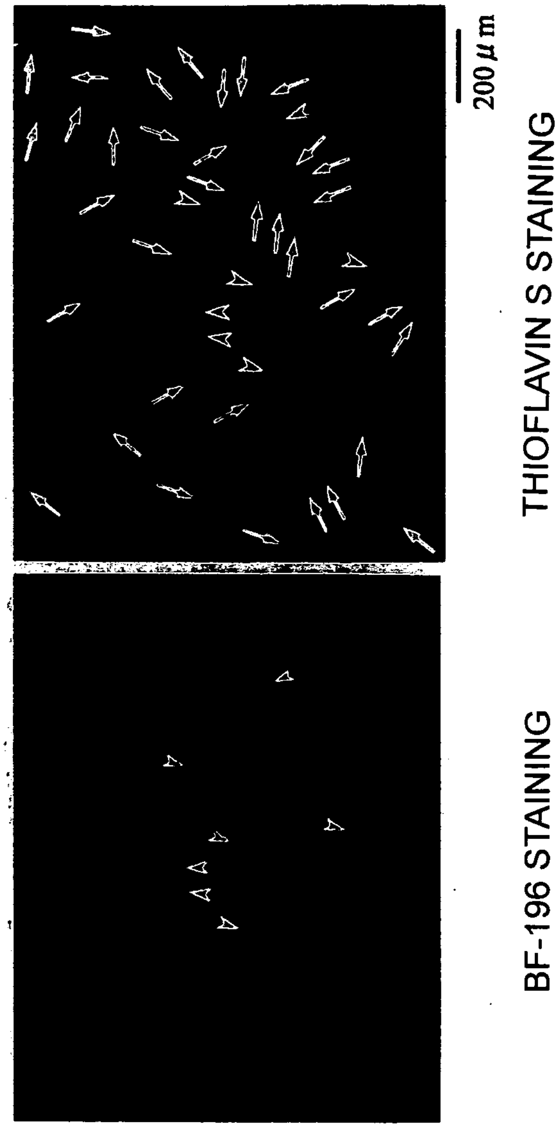

[FIG. 8]

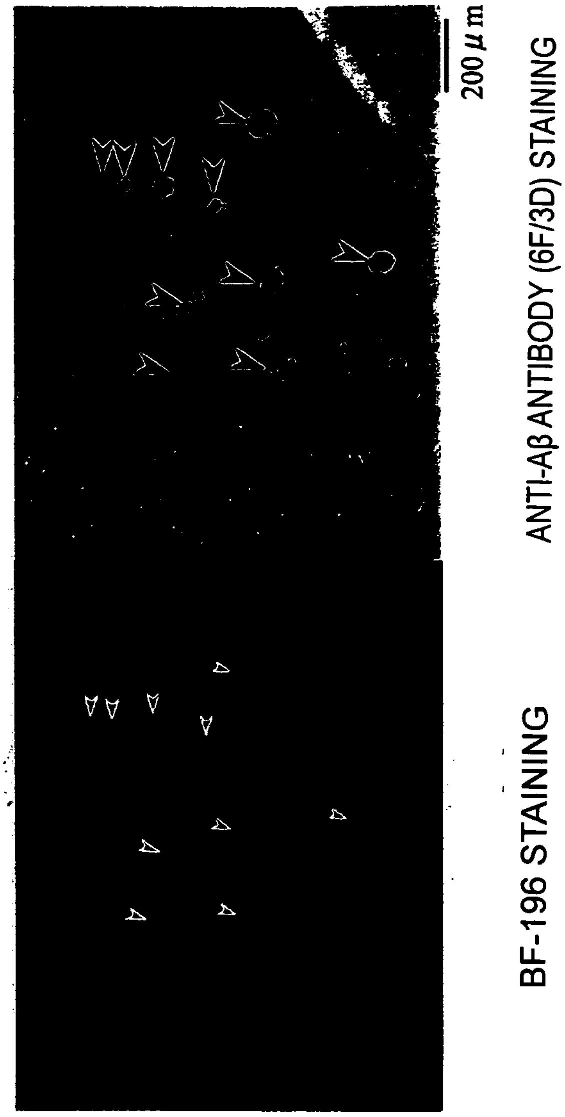

[FIG. 9]

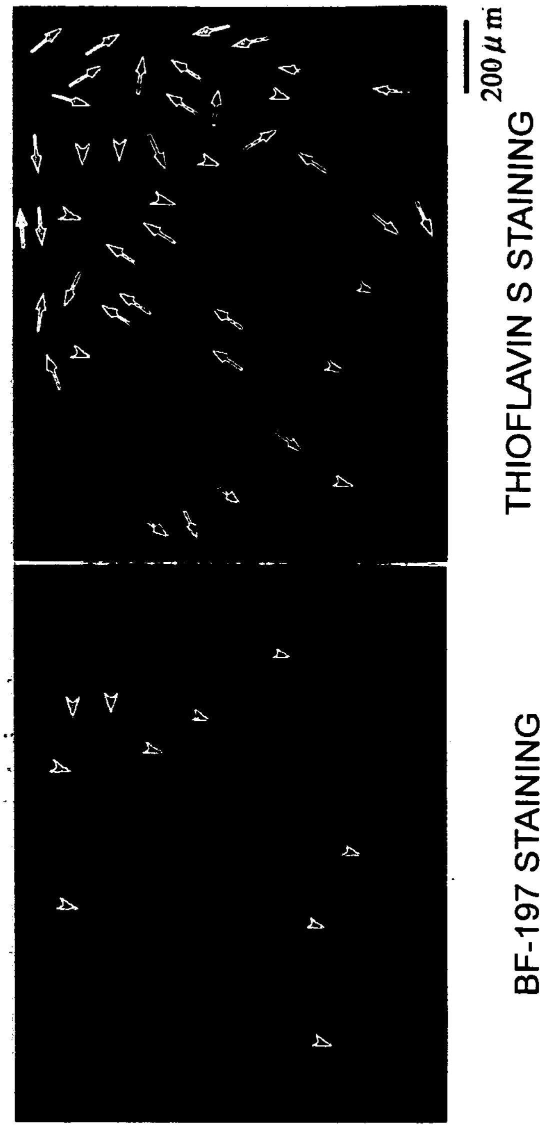

[FIG. 1 0]

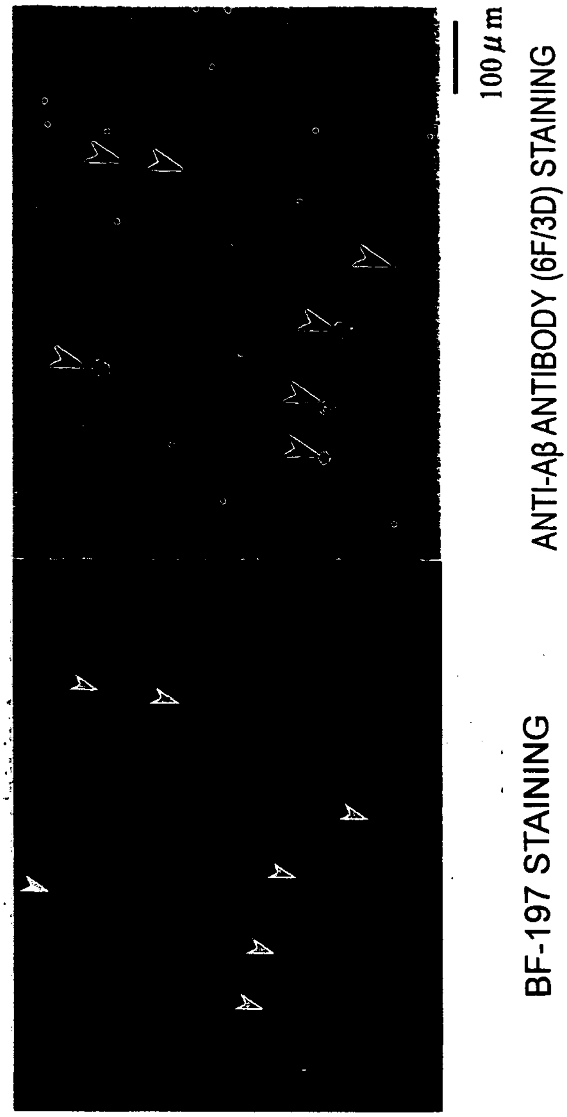

[FIG. 1 1]

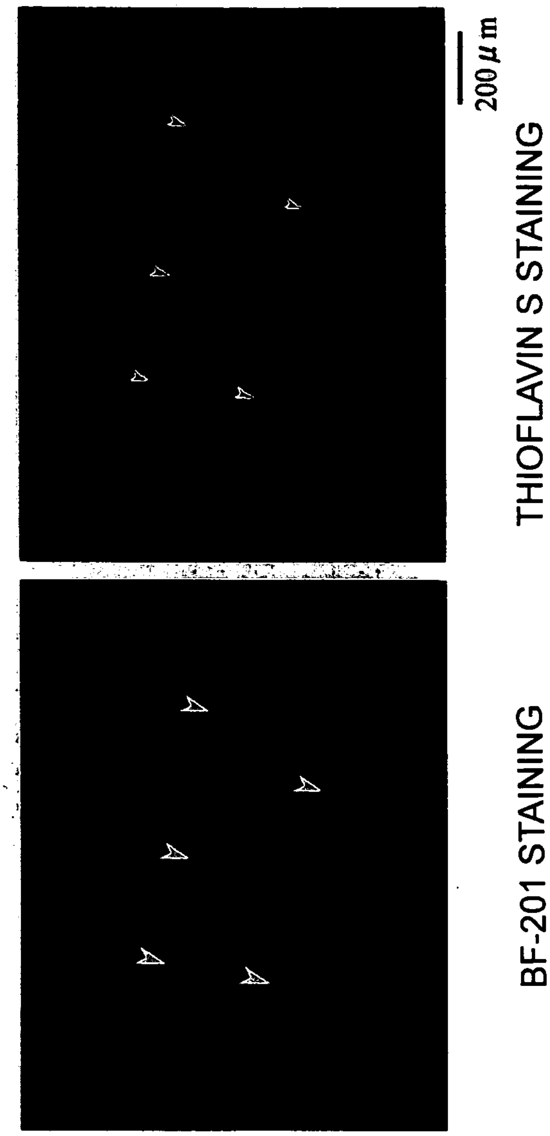

[FIG. 1 2]

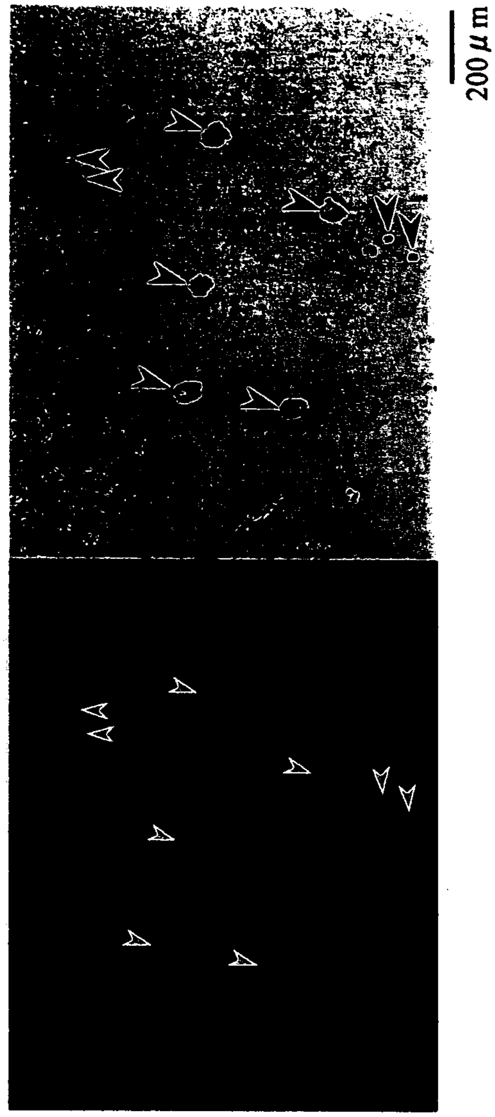


ANTI-Aβ ANTIBODY (6F/3D) STAINING

BF-201 STAINING

[FIG. 1 3]

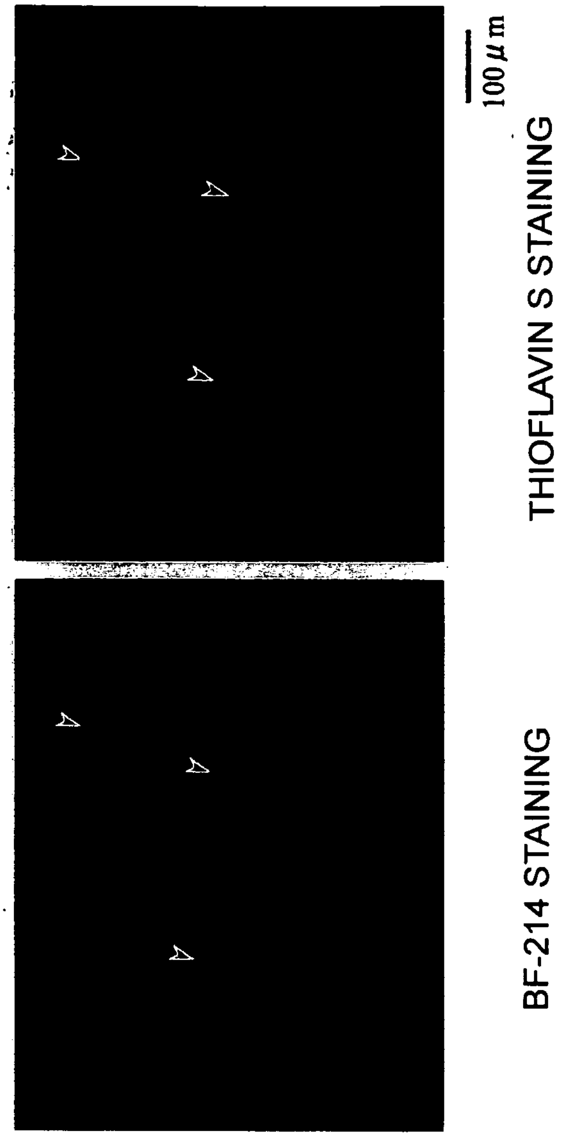

[FIG. 1 4]

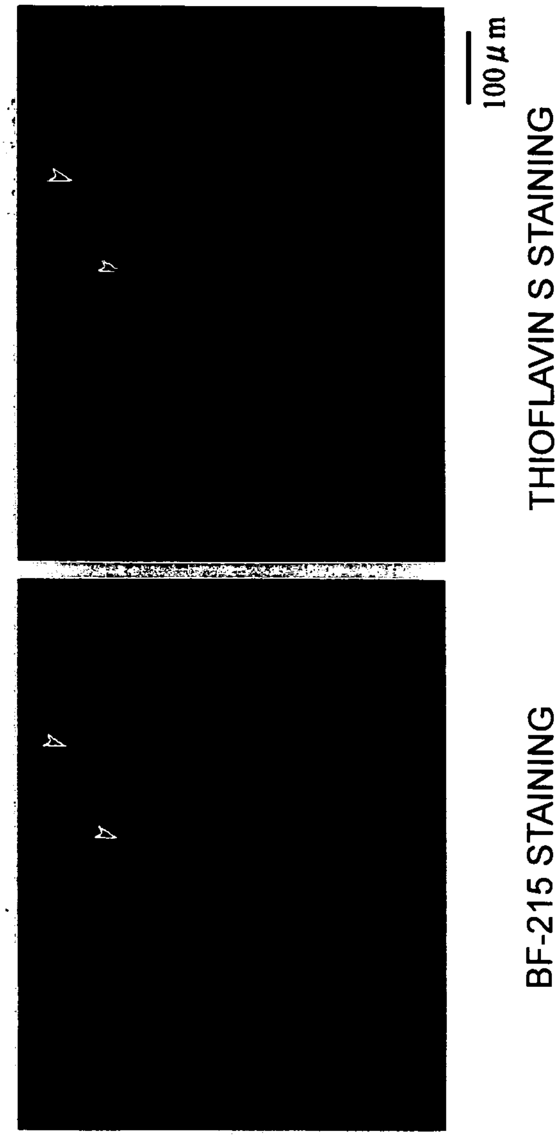

[FIG. 15]

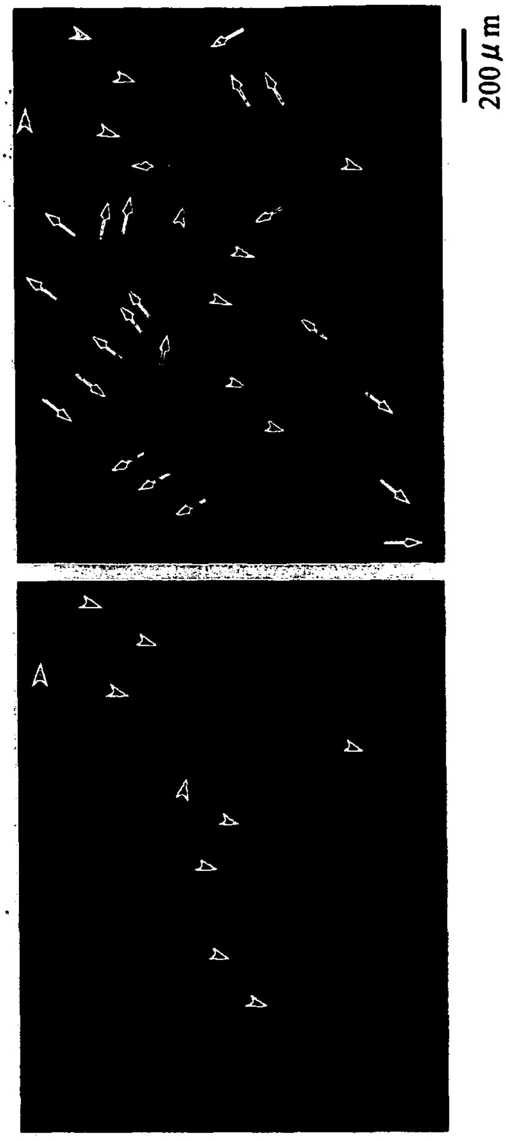


THIOFLAVIN S STAINING

BF-227 STAINING

[FIG. 16]

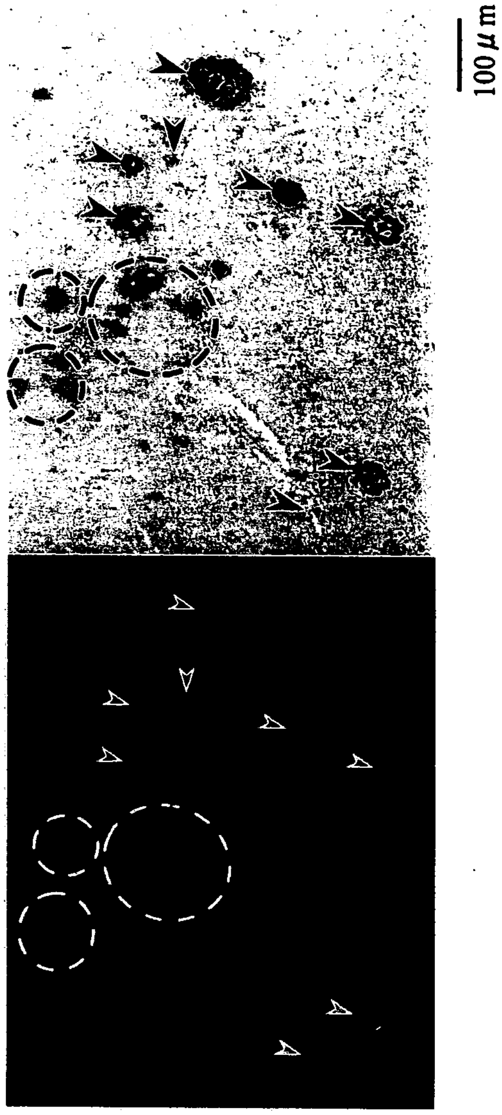


ANTI-Aβ ANTIBODY (6F/3D) STAINING

BF-227 STAINING

[FIG. 1 7]

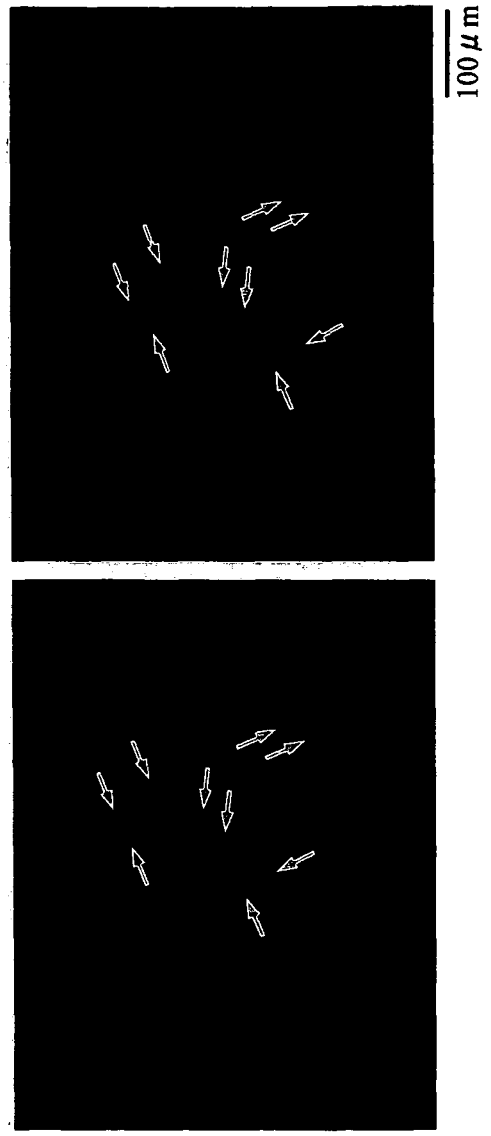


THIOFLAVIN S STAINING

BF-221 STAINING

[FIG. 1 8]

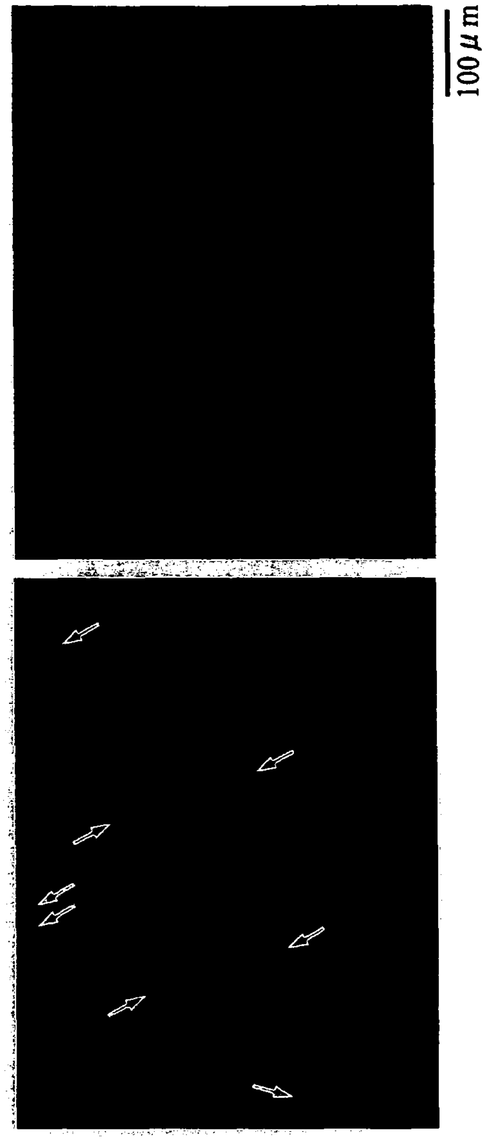


ANTI-PHOSPHORYLATED TAU ANTIBODY(pSer422) STAINING

BF-221 STAINING

[FIG. 19]

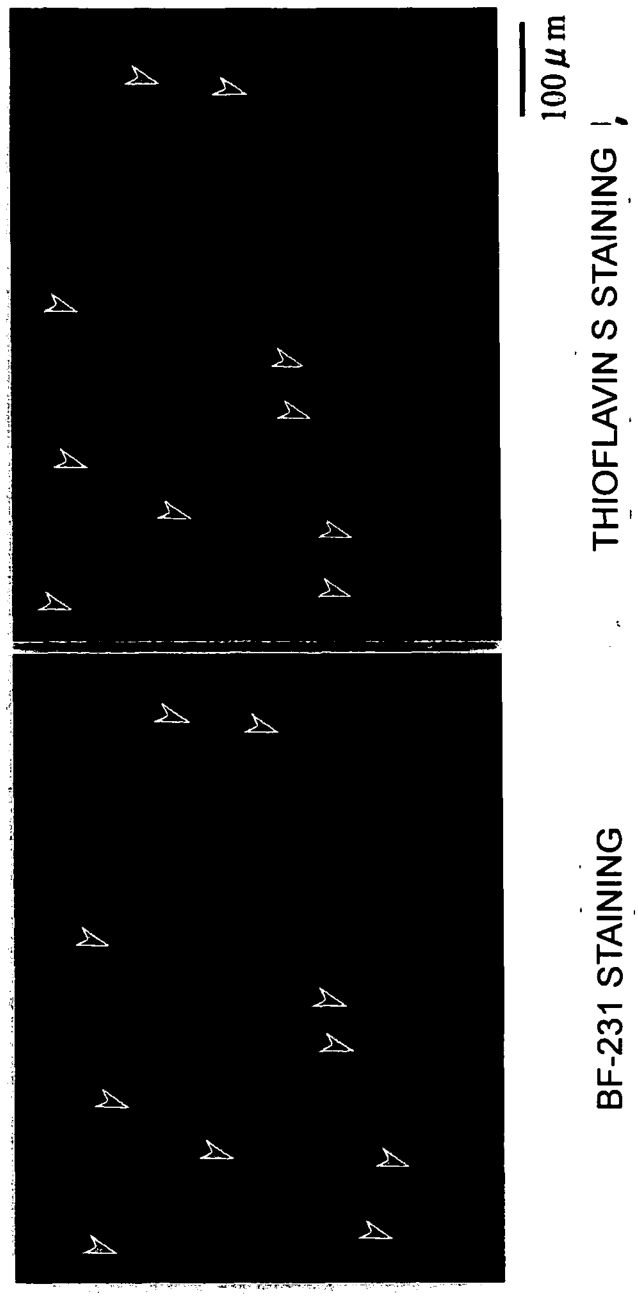

**INTERNATIONAL SEARCH REPORT**

International application No.
PCT/JP2004/011546

A. CLASSIFICATION OF SUBJECT MATTER
Int.Cl[7] C07D215/12, C07D235/06, C07D277/64, C07D277/42, C07D263/57, C07D263/48, C07D263/32, C07D417/06, C07D413/04, C07D417/04, C07D403/06, A61K51/04, A61K31/47, A61K31/4184, A61K31/426,

According to International Patent Classification (IPC) or to both national classification and IPC

B. FIELDS SEARCHED

Minimum documentation searched (classification system followed by classification symbols)
Int.Cl[7] C07D215/12, C07D235/06, C07D277/64, C07D277/42, C07D263/57, C07D263/48, C07D263/32, C07D417/06, C07D413/04, C07D417/04, C07D403/06, A61K51/04, A61K31/47, A61K31/4184, A61K31/426,

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| E,A | JP 2004-250407 A (BF Research Institute, Inc.), 09 September, 2004 (09.09.04), Full text; Registry Nos. 682763-59-3, 748817-30-3 (Family: none) | 1-18,21,23, 25-27,33 |
| P,A | JP 2004-67659 A (BF Research Institute, Inc.), 04 March, 2004 (04.03.04), Full text; Registry No. 666826-27-3 (Family: none) | 1-18,21,23, 25-27,33 |
| P,A | WO 03/106439 A1 (BF RESEARCH INSTITUTE INC.), 24 December, 2003 (24.12.03), Full text (Family: none) | 1-18,21,23, 25-27,33 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 November, 2004 (09.11.04) | 07 December, 2004 (07.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/011546

C (Continuation). DOCUMENTS CONSIDERED TO BE RELEVANT

| Category* | Citation of document, with indication, where appropriate, of the relevant passages | Relevant to claim No. |
|---|---|---|
| P,A | Chem.Abstr., (2004) Vol.141, abstract No. 19735, abstract Registry Nos. 3224-80-4, 13242-17-6, 24675-13-6, 36772-53-9, 634911-43-6, 634911-46-9, 634911-47-0, 634911-48-1, 634911-50-5, 698398-23-1, 698398-24-2, 698398-25-3, 698398-26-4, & OKUMURA, N. et al., "Styrylbenzeoxazole derivatives for in vivo imaging of amyloid plaques in the brain.", Journal of Neuroscience, 10 March, 2004 (10.03.04), Vol.24, No.10, pages 2535 to 2541 | 1-18,21,23, 25-27,33 |
| A | WO 02/16333 A2 (UNIVERSITY OF PITTSBURGH), 28 February, 2002 (28.02.02), Full text; Registry Nos. 84645-69-2, 86912-93-8, 101645-25-4, 401813-33-0, 144528-14-3 & AU 2001/86702 B & EP 1334091 A2 & JP 2004-506723 A | 1-18,21,23, 25-27,33 |
| A | KUNG H. et al., "Nobel stilbenes as probes for amyloid plaques.", Journal of the American Chemical Society, (2001), Vol.123, No.50, pages 12740 to 12741, full text, abstract, Registry No. 1628-58-6 | 1-18,21,23, 25-27,33 |
| A | WO 03/18070 A1 (TRUSTEES OF THE UNIVERSITY OF PENNSYLVANIA), 06 March, 2003 (06.03.03), Full text; Registry Nos. 190079-27-7, 499786-91-3, 499786-92-4 & US 2003/149250 A & EP 1432453 A1 | 1-18,21,23, 25-27,33 |
| A | BOHRMANN B. et al., "Self-assembly of β-amyloid 42 is retarded by small molecular ligands at the stage of structural in termediates.", Journal of Structural Biology, (2000), Vol.130, No.(2-3), pages 232 to 246, Full text, Registry No. 319917-07-2 | 1-18,21,23, 25-27,33 |
| A | JP 2003-522174 A (Bristol-Myers Squibb Co.), 22 July, 2003 (22.07.03), Full text & WO 01/57034 A1 & EP 1268472 A1 & CA 2399274 A1 & US 2001/47019 A & US 2003/18058 A | 1-18,21,23, 25-27,33 |

Form PCT/ISA/210 (continuation of second sheet) (January 2004)

**INTERNATIONAL SEARCH REPORT**

International application No.
PCT/JP2004/011546

C (Continuation). DOCUMENTS CONSIDERED TO BE RELEVANT

| Category* | Citation of document, with indication, where appropriate, of the relevant passages | Relevant to claim No. |
|---|---|---|
| X | JP 2002-518380 A (Bristol-Myers Squibb Co.), 25 June, 2002 (25.06.02), Full text Claims 1, 16 to 19; Par. No. [0031]; page 65, examples, No.54, Registry Nos. 252662-38-7, 252661-03-3 & WO 99/65884 A1 & US 6407124 A & CA 2332325 A1 & AU 9944311 B & EP 1087951 A1 & US 2002/165259 A | 1-3,15,16, 21,25,27 |
| X | JP 58-182640 A (Hitachi, Ltd.), 25 October, 1983 (25.10.83), Registry No. 10004-83-8 & EP 92255 A1 & US 4619879 A | 1,2,25 |
| X | JP 2-28175 A (Sawai Pharmaceutical Co., Ltd.), 30 January, 1990 (30.01.90), Registry Nos. 123970-79-6, 123970-79-6 & EP 321115 A1 & US 4946855 A | 1,2,17,25 |
| X | Chem. Abstr., (1999) Vol.132, abstract No. 293653, abstract Registry No. 264599-77-1 & KASSEM E M M "Synthesis fo some new 2,4-disubstituted quinoline derivatives with expected biological activity." Egyptian Journal of Chemistry, (1999) Vol.42, No.4, pages 413 to 419 | 1,2,17,25 |

Form PCT/ISA/210 (continuation of second sheet) (January 2004)

**INTERNATIONAL SEARCH REPORT**

International application No.
PCT/JP2004/011546

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 19, 20, 22, 24, 28-31
because they relate to subject matter not required to be searched by this Authority, namely:
Claims 19, 20, 22, 24, 28 to 31 involve embodiments concerning methods for treatment of the human body by therapy or diagnostic methods and thus relate to a subject matter which this International Searching Authority is not required, (continued to extra sheet.)

2. ☒ Claims Nos.: 32, 34, 35
because they relate to parts of the international application that do not comply with the prescribed requirements to such an extent that no meaningful international search can be carried out, specifically:
Since the scopes of the "precursor compound" relating to the inventions according to claims 32, 34 and 35 are not sufficiently and clearly specified in individual claims, no meaningful international search can be made thereon.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.
PCT/JP2004/011546

Continuation of A. CLASSIFICATION OF SUBJECT MATTER
(International Patent Classification (IPC))

Int.Cl$^7$ A61K31/428, A61K31/423, A61K31/422, A61K31/421, A61K31/427, A61K31/4709, A61K31/433, A61K31/4245, A61P25/28

(According to International Patent Classification (IPC) or to both national classification and IPC)

Continuation of B. FIELDS SEARCHED
Minimum documentation searched (International Patent Classification (IPC))

Int.Cl$^7$ A61K31/428, A61K31/423, A61K31/422, A61K31/421, A61K31/427, A61K31/4709, A61K31/433, A61K31/4245, A61P25/28

Minimum documentation searched (classification system followed by classification symbols)

Continuation of Box No.II-1 of continuation of first sheet(2)

under the provisions of Article 17(2)(a)(i) of the PCT and Rule 39.1(iv) of the Regulations under the PCT, to search.

<Subject of search>

The formula I as set forth in claim 1 includes a great number of compounds. However, only part of the claimed compounds are specifically presented in the description or the drawings. Further, the efficacies as the active ingredient of compositions for diagnosing diseases with amyloid β-protein accumulation or "conformation diseases" have been confirmed based on experimental data concerning only part of the specifically presented compounds as described above.

Therefore, the search on prior art was made exclusively on the compounds of the formula I wherein the D and E-containing ring is a single ring (i.e., $R_4$ and $R_5$ not forming together a benzene ring), specific examples of which are cited, and the compounds the data of which proving the staining properties for senile plaque or neurofibrillary changes thereof in practice (i.e., the compounds wherein D is O and E is N; forming a single oxazole ring) are presented in the description or the drawings. Moreover, the search on prior art was carried out mainly on compounds having been known as relating to diseases with amyloid β-protein accumulation typified by Alzheimer's disease.

Form PCT/ISA/210 (extra sheet) (January 2004)